(19)

Europäisches
Patentamt
European
Patent Office
Office européen
des brevets

(11)　**EP 4 260 826 A1**

(12)　**EUROPEAN PATENT APPLICATION**
published in accordance with Art. 153(4) EPC

(43) Date of publication:
**18.10.2023　Bulletin 2023/42**

(21) Application number: **21901983.3**

(22) Date of filing: **17.06.2021**

(51) International Patent Classification (IPC):
*A61B 17/80* (2006.01)　　　*A61L 31/02* (2006.01)
*A61L 31/06* (2006.01)　　　*A61L 31/14* (2006.01)

(86) International application number:
**PCT/CN2021/100712**

(87) International publication number:
**WO 2022/121271 (16.06.2022 Gazette 2022/24)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **11.12.2020　CN 202011460429**

(71) Applicants:
- **Chinese PLA General Hospital
  Beijing 100039 (CN)**
- **Beijing Chunlizhengda Medical Instruments Co.,
  Ltd
  Beijing 101112 (CN)**

(72) Inventors:
- **PENG, Jiang
  Beijing 100039 (CN)**
- **WANG, Zhenguo
  Beijing 101112 (CN)**
- **MENG, Haoye
  Beijing 100039 (CN)**
- **LU, Qiang
  Beijing 100039 (CN)**
- **PAN, Chao
  Beijing 101112 (CN)**
- **WANG, Aiyuan
  Beijing 100039 (CN)**
- **ZHOU, Hao
  Beijing 101112 (CN)**
- **XU, Wenjing
  Beijing 100039 (CN)**
- **LIU, Wei
  Beijing 101112 (CN)**
- **ZHANG, Xingyan
  Beijing 101112 (CN)**
- **SHI, Chunbao
  Beijing 101112 (CN)**
- **CHENG, Xi
  Beijing 101112 (CN)**
- **YUE, Shujun
  Beijing (CN)**

(74) Representative: **Zaboliene, Reda
  Metida
  Business center Vertas
  Gyneju str. 16
  01109 Vilnius (LT)**

(54) **FRACTURE REPAIR DEVICE CAPABLE OF ACHIEVING TRANSITION FROM MECHANICAL
OSTEOSYNTHESIS (AO) TO BIOLOGICAL OSTEOSYNTHESIS (BO)**

(57)　The present invention discloses a fracture repair device realizing transition from mechanical fixation (association of osteosynthesis, AO) to biological fixation (biological osteosynthesis, BO), including: a bone repair instrument, wherein auxiliary components are arranged at positions, close to a repaired bone surface, of the bone repair instrument, and each auxiliary component is made of a degradable metal material or a composite material thereof or a degradable polymeric material. The device of the present invention only has the advantages of AO rigid mechanical fixation, but also can effectively improve the defects of bone disconnection, fixed segment osteoporosis, re-fracture after defixation, etc., frequently occurring under AO rigid mechanical fixation, realizing transition from mechanical fixation (association of osteosynthesis, AO) to biological fixation (biological osteosynthesis, BO) during bone fixation or repair.

FIG. 1

Processed by Luminess, 75001 PARIS (FR)

EP 4 260 826 A1

**Description**

The present invention claims the priority of Chinese patent application CN202011460429.0, the contents described in the description, the drawings and the claims of this priority document are incorporated in the description of the present invention in their entirety, and are taken as part of the original description of the present invention. The applicant further states that the applicant has the right to amend the description and claims of the present invention based on this priority document.

**TECHNICAL FIELD**

The present invention relates to the technical field of bone fixation and repair medical devices, in particular to a fracture repair device realizing transition from mechanical fixation (association of osteosynthesis, AO) to biological fixation (biological osteosynthesis, BO).

**BACKGROUND**

Internal fixation always plays an important role in the treatment of fractures. For more than 60 years, rigid compressive internal fixation advocated by mechanical fixation (association of osteosynthesis, AO) has been dominant in fracture treatment, and its technical core is compressive fixation between fracture ends and reconstruction of an anatomical structure, so as to achieve the purpose of early limb movement. Mechanical fixation (association of osteosynthesis, AO) has now formed a complete system from a theory, a principle, and a method to a device, and equipment, and has become one of the classical means in the field of fracture treatment today.

With advances in the medical level, mechanical fixation (association of osteosynthesis, AO) scholars reflected the scientific nature of its fixation principle, and proposed a new concept of biological fixation (biological osteosynthesis, BO): emphasizing maximum protection of blood supply at a fracture site, not sacrificing too much soft tissue pedicles for anatomical reduction, using internal fixation instruments with low elastic modulus and good biocompatibility, reducing a contact surface between an internal fixator and fixed bone, etc. Biological fixation (biological osteosynthesis, BO) is a new concept in development, not a mature system, although it has advantages in theory, the corresponding supporting methods, devices, and instruments are not complete, there are generally problems in application such as fewer cases, lack of controls, and long surgery time; surgical subjects are limited to the femur and tibia of lower limbs; and requirements for operators, intraoperative X-ray fluoroscopy and instruments are also relatively high, etc. This not only involves the development of a medical theory and an orthopedic surgical technique, but also is closely related to the advancement of the detection means and biomedical materials. In recent years, with the continuous development of biomedical materials, biomedical degradable metals and degradable high molecular materials have become potential materials for biological fixation devices due to their good biosafety, degradability, and elastic moduli matching with human bone. There are three main types of degradable metal materials currently recognized by academia: degradable magnesium-based materials, degradable iron-based materials, and degradable zinc-based materials that have been extensively studied in recent years. Magnesium alloys have been studied in the biomedical field for over a hundred years. The material density (1.74 $g/cm^3$) is close to that of human bone (about 1.75 $g/cm^3$), while the magnesium alloy also has an elastic modulus similar to that of the human bone, greatly reducing the "stress shielding" effect. Moreover, magnesium, as a nutrient element, is widely involved in various physiological activities in the human body, and studies have shown that a suitable concentration of magnesium ions is beneficial for inducing the differentiation and growth of bone cells. The magnesium element is abundant in reserves in China and has great economic advantages. Iron and iron alloys have more excellent mechanical properties and slower degradation rates than magnesium alloys, and an iron element is a main functional element of hemoglobin in a human body, undertaking important physiological activities in the human body. In addition to having good biosafety, zinc and zinc alloys have superior mechanical properties over magnesium alloys and corrosion degradation properties between magnesium and iron, and have also become hot-spot materials for scholars to study. The application as a bone fixation repair material is relatively mature compared with degradable metals, and a variety of manufactured bone repair instruments are currently marketed. With the gradual improvement of a chemical preparation process, the defects in artificial synthesis are gradually reduced, artificial polymer materials have obvious degradability and biological activity compared with natural polymer materials, and are also superior to the natural polymer materials in mechanical properties and controllable degradation rates.

Because the existing mechanical fixation (association of osteosynthesis, AO) technology excessively pursues the mechanical stability of a fixation system, it does not pay attention to the biological characteristics of bone. For example, due to the pursuit of rigid internal fixation, especially comminuted and complex fractures, in order to achieve rigid fixation between fracture segments, it is sometimes necessary to carry out extensive peeling, which destroys the peripheral blood supply, and postoperative complications such as delayed union or nonunion of fractures, infection, osteoporosis of fixed segments and so on often occur. Moreover, this type of direct healing or primary healing is not firm, and fracture often occurs again after taking out a steel plate.

**SUMMARY**

The present invention provides a fracture repair device realizing transition from mechanical fixation (association of osteosynthesis, AO) to biological fixation (biological osteosynthesis, BO), and solves the problems raised in the background that postoperative complications such as delayed union or nonunion of fractures, infection, osteoporosis of fixed segments and so on often occur after treatment with the existing mechanical fixation (association of osteosynthesis, AO) technology, and fracture is easy to occur again after taking out the steel plate.

In order to achieve the above object, the present invention provides the following technical solution: a fracture repair device realizing transition from mechanical fixation (association of osteosynthesis, AO) to biological fixation (biological osteosynthesis, BO) includes a bone repair instrument, wherein auxiliary components are arranged at positions, close to a repaired bone surface, of the bone repair instrument, and each auxiliary component is made of a degradable material.

Preferably, the degradable material includes a degradable metal material or a composite material thereof or a degradable polymeric material, and the bone repair instrument may be made of biomedical stainless steel, pure titanium, Ti6A14V or Ti6Al7Nb; and

the degradable metal material or the composite material thereof includes any one of magnesium or a magnesium alloy or a magnesium-based composite, zinc or a zinc alloy or a zinc-based composite, and iron or an iron alloy or an iron-based composite.

Preferably, the magnesium or magnesium alloy or magnesium-based composite includes any one of high purity magnesium, Mg-Zn, Mg-Sr, Mg-Ca, Mg-Li, Mg-Y, Mg-Zn-Ca, WE43, AZ31B, AZ91, HA/Pure Mg, HA/Mg-Zn, HA/Mg-Ca, HA/Mg-Zn-Ca, $\beta$-TCP/Pure Mg, $\beta$-TCP/Mg-Zn, $\beta$-TCP/Mg-Ca, $\beta$-TCP/Mg-Zn-Ca, MgO/Pure Mg, MgO/Mg-Zn, and MgO/Mg-Ca;

the zinc or zinc alloy or zinc-based composite includes any one of high purity zinc, Zn-Mg, Zn-Cu, Zn-Ca, Zn-Li, Zn-Y, Zn-Sr, HA/Pure Zn, HA/Zn-Mg, HA/Zn-Cu, HA/Zn-Ca, $\beta$-TCP/Pure Zn, $\beta$-TCP/Zn-Mg, $\beta$-TCP/Zn-Cu, $\beta$-TP/Zn-Ca, ZnO/Pure Zn, and ZnO/Zn-Mg;

the iron or iron alloy or iron-based composite includes any one of high purity iron, Fe-X, Fe-Mn-Si, Fe-Mn-C, Fe-Mn-Pd, CNT/Fe, $Fe_2O_3$/Fe, HA/Fe, and $\beta$-TCP/Fe, wherein X is any one of Mn, Co, Al, W, Pt, Ag, Sn, B, C, and S; and

the degradable polymeric material includes any one of polylactic acid, a copolymer, polycaprolactone, polydioxanone, polyhydroxyalkanoate, polytrimethylene carbonate, polyurethane, and polyether urethane.

Preferably, the auxiliary components include any one or more of a pad, a plate and a block, wherein the pad, the plate, and the block include any one or more of a circular ring-shaped gasket, a plate-shaped washer, and a square cushion block.

Preferably, the surface of each auxiliary component is subjected to surface treatment including any one of micro-arc oxidation, chemical deposition, and anodization.

Preferably, a connection mode between each auxiliary component and the bone repair instrument includes direct contact connection or pin connection.

The fracture repair device realizing transition from mechanical fixation (association of osteosynthesis, AO) to biological fixation (biological osteosynthesis, BO) of the present invention has the following obvious advantages: 1) the AO rigid fixation system involved is a set of maturely applied bone repair methods, which has been proved to have great advantages in reliability and repair performance in long-term practice. And the numerous categories of repair equipment that are ready-made with the AO system can achieve the improvement method described in this design without too much improvement.

2) The added raw materials of the devices such as the pad, the plate, and the block are biomedical degradable materials, have good biocompatibility and degradability, and have elastic moduli closer to human bone, so that "stress shielding" effects can be avoided well.

3) The contact fit between the devices such as the pad, the plate, and the block and a universal metal bone repair material includes direct contact and pin fit. Wherein the direct contact is to directly place a degradable metal pad or plate between the instrument and the fixed repaired bone surface to be subjected to compression fixation by passing through through holes of the pad or plate through bolts; and the pin fit is primarily directed to the block device, and the block device is directly inserted into the instrument through a protrusion on the block or the block device and the instrument are locked through a taper fit.

4) The surface of the pad, plate or block achieves effective regulation of a degradation rate or induces improvement of the osteogenic performance by means of micro-arc oxidation, chemical soaking or anodization.

The fracture repair device realizing transition from mechanical fixation (association of osteosynthesis, AO) to biological fixation (biological osteosynthesis, BO) is simple in design and preparation method, low in cost, high in safety factor, and beneficial to industrial production.

Additional features and advantages of the present invention will be set forth in the subsequent description, and in part will be obvious from the description, or will be understood by the implementation of the present invention. The objectives and other advantages of the present invention may be achieved and obtained by the devices specifically pointed out in the written description and drawings.

The technical solution of the present invention is further described in detail below by the accompanying drawings and embodiments.

## BRIEF DESCRIPTION OF THE DRAWINGS

The drawings are used to provide a further understanding of the present invention and constitute part of the description, which are used to interpret the present invention together with the embodiments of the present invention, and do not constitute limitations on the present invention. In the drawings:

FIG. 1 is a schematic diagram of a first connection of a fracture repair device of the present invention;
FIG. 2 is a front view of the first connection of the fracture repair device of the present invention;
FIG. 3 is a cross-sectional view of the first connection of the fracture repair device of the present invention;
FIG. 4 is a schematic diagram of a second connection of a fracture repair device of the present invention;
FIG. 5 is a front view of the second connection of the fracture repair device of the present invention;
FIG. 6 is a cross-sectional view of the second connection of the fracture repair device of the present invention;
FIG. 7 is a schematic diagram of a third connection of a fracture repair device of the present invention;
FIG. 8 is a front view of the third connection of the fracture repair device of the present invention;
FIG. 9 is a cross-sectional view of the third connection of the fracture repair device of the present invention;
FIG. 10 is a schematic diagram of a mounting device of the fracture repair device of the present invention;
FIG. 11 is a schematic structural diagram of one embodiment of a preprocessing component of the present invention;
FIG. 12 is a schematic enlarged partial view of A in FIG. 11;
FIG. 13 is a first diagram of one experiment of the present invention;
FIG. 14 is a second diagram of one experiment of the present invention;
FIG. 15 is a third diagram of one experiment of the present invention;
FIG. 16 is a first diagram of another experiment of the present invention;
FIG. 17 is a second diagram of another experiment of the present invention;
FIG. 18 is a third diagram of another experiment of the present invention;
FIG. 19 is a fourth diagram of another experiment of the present invention; and
FIG. 20 is a fifth diagram of another experiment of the present invention.

As follows: 1, bone repair instrument; 2, auxiliary component; 3, circular ring-shaped gasket; 4, plate-shaped washer; 5, square cushion block; 6, first screw; 7, first rocker; 8, moving plate; 9, roller; 10, guide plate; 11, baffle; 12, first spring; 13, first rotary shaft; 14, gear; 15, first rack; 16, first clamp plate; 17, second spring; 18, second rack; 19, second clamp plate; 20, third spring; 21, second screw; 22, second rocker; 23, pressing plate; 24, fixed column; 30, pretreatment device; 301, fixed base; 302, cam bracket; 303, cam; 304, first slide rail; 305, first sliding block; 306, first electric telescopic rod; 307, first connecting rod; 308, vertical bracket; 309, horizontal mounting plate; 3010, fixed pulley; 3011, second slide rail; 3012, second sliding block; 3013, connecting line; 3014, first connecting plate; 3015, placement box; 3016, second vertical connecting rod; 3017, pretreatment spray head; 3018, connecting block; 3019, fourth spring; 3020, fifth spring; 3021, third connecting rod; 3022, housing; 3023, third sliding block; 3024, sixth spring; 3025, first processing plate; 3026, first processing component; 30261, hollow fixed connecting block; 30262, sliding rod; 30263, vertical connecting plate; 30264, disk body; 30265, seventh spring; 30266, processing block; 30267, second electric telescopic rod; 30268, eighth spring; and 3027, fourth connecting rod.

## DETAILED DESCRIPTION

Preferred embodiments of the present invention will be described below with reference to the accompanying drawings. It should be understood that the preferred embodiments described herein are merely illustrative and explanatory of the present invention and are not intended to limit the present invention.

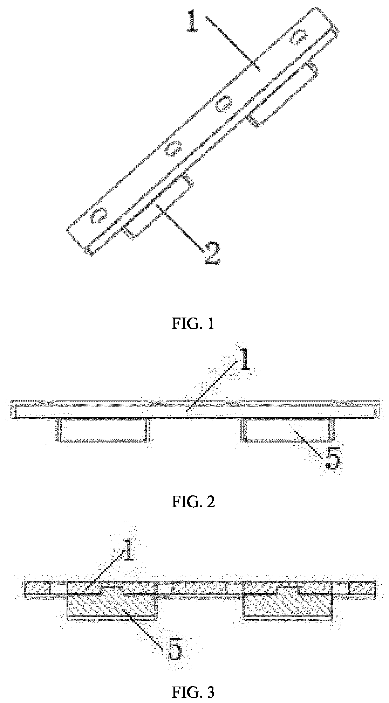

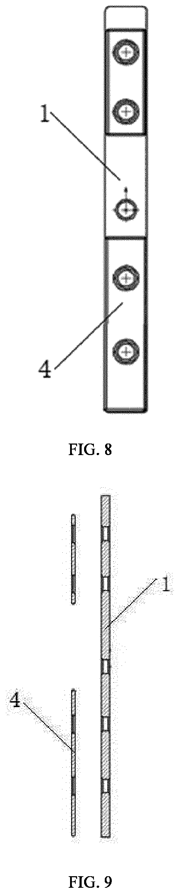

An embodiment of the present invention provides a fracture repair device realizing transition from mechanical fixation (association of osteosynthesis, AO) to biological fixation (biological osteosynthesis, BO), as shown in FIGS. 1-9, including a bone repair instrument 1 (which may be a metal bone repair instrument), wherein auxiliary components 2 are arranged at positions, close to a repaired bone surface (i.e. a surface of bone to be repaired), of the bone repair instrument 1, and each auxiliary component 2 is made of a degradable material or a composite material thereof or a degradable polymeric material.

Preferably, there is a clearance distance between the bone repair instrument 1 and the repaired bone surface, and the auxiliary components 2 are in contact with the repaired bone surface.

Preferably, the bone repair instrument 1 may be a universal bonesetting plate; and the bone repair instrument 1 may be made of biomedical stainless steel, pure titanium, Ti6A14V or Ti6Al7Nb or the like. The bone repair instrument 1 is a bonesetting plate, and the bone repair instrument 1 may be made of biomedical stainless steel, pure titanium, Ti6A14V, or Ti6Al7Nb or the like, and the above materials have good mechanical properties, good corrosion resistance, and good biocompatibility, and can be implanted in a human body for easy fixation of fracture sites.

A working principle and beneficial effects of the above technical solution are as follows: the bone repair instrument 1 is first selected, the bone repair instrument 1 is adapted to the existing

AO rigid fixation system, the AO rigid fixation system is a set of maturely applied bone repair systems/methods, which has been proved to have great advantages in reliability and repair performance in long-term practice, and the numerous categories of repair equipment that are ready-made with the AO rigid fixation system can achieve the improvement method described in this design without too much improvement, the auxiliary components 2 are then selected, each auxiliary component 2 is made of a degradable metal or a composite material thereof or a degradable polymeric material, the auxiliary components 2 are connected with the bone repair instrument 1 in a set manner to form a repair component, the repair component is finally mounted on repaired bone, the surface of each auxiliary component 2 is in contact with the repaired bone surface during mounting, in the present invention, the auxiliary components 2 made of the degradable metal or the composite material thereof or the degradable polymeric material are placed at sites, which are in contact with the repaired bone surface, of the bone repair instrument 1, and are in direct contact with the repaired bone surface, by means of good biocompatibility and degradable properties of the auxiliary components 2 made of the degradable metal or the composite material thereof or the degradable polymeric material, as the auxiliary components 2 progressively degrade, a fracture surface at a fixation site will heal progressively, the volumes of the auxiliary components 2 gradually decrease, such that pressure at the fixation site gradually decreases, blood supply at a compressed part can be gradually restored, the nutrient supply and metabolic circulation at a fracture site can be ensured, and the fracture healing speed can be accelerated, and in addition, the auxiliary components 2 made of the degradable metal or the composite material thereof or the degradable polymeric material have a suitable elastic modulus, which can better transmit a load and avoid the "stress shielding" effect, and the reaction force of the auxiliary components 2 will well stimulate the functional differentiation of fixed cells on the repaired bone surface which is in contact with the auxiliary components 2, so that the healing situation of repaired bone is more ideal, and this method not only has the advantages of AO rigid mechanical fixation, but also can effectively improve the defects of bone disconnection, fixed segment osteoporosis, re-fracture after defixation, etc., frequently occurring under AO rigid mechanical fixation, realizing transition from mechanical fixation (association of osteosynthesis, AO) to biological fixation (biological osteosynthesis, BO) during bone fixation or repair.

Embodiment 2, on the basis of Embodiment 1, the degradable metal or the composite material thereof or the degradable polymeric material mainly includes any one of magnesium or a magnesium alloy or a magnesium-based composite, zinc or a zinc alloy or a zinc-based composite, iron or an iron alloy or an iron-based composite, and a degradable polymeric material.

A working principle and beneficial effects of the above technical solution are as follows: the magnesium or the magnesium alloy or the magnesium-based composite, the zinc or the zinc alloy or the zinc-based composite, the iron or the iron alloy or the iron-based composite, and the degradable polymeric material can be gradually degraded when placed in a human body, so that the volumes of the auxiliary components 2 are gradually reduced, the blood supply at a compressed part can be gradually restored to ensure nutrient supply and metabolic circulation at a fracture site, and the degraded substances are easily absorbed by the human body without damaging the human health.

Embodiment 3, on the basis of Embodiment 2

The magnesium or magnesium alloy or magnesium-based composite includes any one of high purity magnesium, Mg-Zn, Mg-Sr, Mg-Ca, Mg-Li, Mg-Y, Mg-Zn-Ca, WE43, AZ31B, AZ91, HA/Pure Mg, HA/Mg-Zn, HA/Mg-Ca, HA/Mg-Zn-Ca, $\beta$-TCP/Pure Mg, $\beta$-TCP/Mg-Zn, $\beta$-TCP/Mg-Ca, $\beta$-TCP/Mg-Zn-Ca, MgO/Pure Mg, MgO/Mg-Zn, and MgO/Mg-Ca;

the zinc or zinc alloy or zinc-based composite includes any one of high purity zinc, Zn-Mg, Zn-Cu, Zn-Ca, Zn-Li, Zn-Y, Zn-Sr, HA/Pure Zn, HA/Zn-Mg, HA/Zn-Cu, HA/Zn-Ca, $\beta$-TCP/Pure Zn, $\beta$-TCP/Zn-Mg, $\beta$-TCP/Zn-Cu, $\beta$-TP/Zn-Ca, ZnO/Pure Zn, and ZnO/Zn-Mg;

the iron or iron alloy or iron-based composite includes any one of high purity iron, Fe-X (X=Mn, Co, Al, W, Pt, Ag, Sn, B, C or S), Fe-Mn-Si, Fe-Mn-C, Fe-Mn-Pd, CNT/Fe, $Fe_2O_3$/Fe, HA/Fe, and $\beta$-TCP/Fe; and

the degradable polymeric material includes any one of polylactic acid (PLA), a copolymer (PLGA), polycaprolactone (PCL), polydioxanone (PDS), polyhydroxyalkanoate (PHA), polytrimethylene carbonate (PTMC), polyurethane (PUR), and polyether urethane (PEU).

A working principle and the beneficial effects of the above technical solution include: the magnesium or magnesium alloy or magnesium-based composite or magnesium alloy-based composite; the iron or iron alloy or iron-based composite or iron alloy-based composite; and the zinc or zinc alloy or zinc-based composite or zinc alloy-based composite. Wherein alloying elements in the magnesium alloy are one or two or more of Zn, Sr, Ca, Li, Y, and RE, wherein the contents by mass of the alloying elements are optionally as follows: 1-10% of Mg, and/or 0.1-0.3% of Sr, and/or 0.1-0.5% of Ca, and/or 0.2-1% of Li, and/or 0.1-5% of Y, and/or 0.01-5% of RE, the balance being pure magnesium, and the magnesium alloy composite is that 0.5-10 vol.% of bioactive ceramic particles $\beta$-TCP or HA or MgO with a particle size of 20 nm to 10 $\mu$m are added into the magnesium alloy, wherein a matrix alloy of the composite is the above magnesium alloy, magnesium, as a nutrient element, is widely involved in various physiological activities in the human body, studies have shown that a suitable concentration of magnesium ions is beneficial for inducing the differentiation and growth of bone cells, and the magnesium element is abundant in reserves in China, and has great economic advantages; alloying elements in the iron alloy are one or two or more of Mn, Co, Al, W, Pt, Ag, Sn, B, C, S, Si, and Pd, wherein the contents by mass of the alloying elements are optionally as follows: 1-40% of Mn, and/or 0.1-3% of Co, and/or 0.1-5% of Al, and/or 0.2-3% of W, and/or 0.1-5% of Pt, and/or 0.1-5% of Ag, and/or 0.1-5% of Sn, and/or 0.01-2% of B, and/or 0.1-10% of C, 0.1-3% of S, 0.1-5% of Si, 0.1-10% of Pd, the balance being pure iron, and the iron alloy composite is that 0.1-10 vol.% of bioactive ceramic particles $\beta$-TCP or HA or $Fe_2O_3$ or CNT with a particle size of 5 nm to 10 $\mu$m are added into the iron alloy, wherein a matrix alloy of the composite is the above iron alloy, the iron and iron alloy have more excellent mechanical properties and slower degradation rate than the magnesium alloy, and the iron element is a main functional element of hemoglobin in a human body, undertaking important physiological activities in the human body; alloying elements in the zinc alloy are one or two or more of Mg, Cu, Ca, Li, Y, and Sr, wherein the contents by mass of the alloying elements are optionally as follows: 1-10% of Mg, and/or 0.1-0.3% of Cu, and/or 0.1-0.5% of Ca, and/or 0.2-1% of Li, and/or 0.1-5% of Sr, and/or 1-3% of Cu, the balance being pure zinc, and the zinc alloy composite is that 1-10 vol.% of bioactive ceramic particles $\beta$-TCP or HA or ZnO with a particle size of 20 nm to 10 $\mu$m are added into the zinc alloy, wherein a matrix alloy of the composite is the above zinc alloy, and in addition to having good biosafety, the zinc and zinc alloy have superior mechanical properties over the magnesium alloy and corrosion degradation properties between magnesium and iron; and the degradable polymeric material is mainly: polylactic acid (PLA), a copolymer (PLGA), polycaprolactone (PCL), polydioxanone (PDS), polyhydroxyalkanoate (PHA), polytrimethylene carbonate (PTMC), polyurethane (PUR) and polyether urethane (PEU), and has significant degradability and biological activity, and is superior to natural polymeric materials in mechanical properties and controllable degradation rates.

Embodiment 4 On the basis of any one of the embodiments 1-3, each auxiliary component 2 may be a circular ring-shaped gasket 3, a plate-shaped washer 4 or a square cushion block 5. A working principle and the beneficial effects of the above technical solution are as follows: each auxiliary component 2 can be in three forms of the circular ring-shaped gasket 3, the plate-shaped washer 4 or the square cushion block 5, and different forms of the auxiliary components 2 can be selected for different fracture positions, different fracture environments and different repair positions, respectively, thereby achieving the best bone repair effect.

Optionally, the connection in the set manner includes direct contact connection or pin connection (insertion or taper contact). The auxiliary components 2 and the bone repair instrument 1 are connected in a set manner, the set manner is direct contact connection or pin connection, when each auxiliary component 2 is the circular ring-shaped gasket 3 or the plate-shaped washer 4, through holes of the circular ring-shaped gasket 3 or the plate-shaped washer 4 are aligned with through holes of a bonesetting plate, the surface of the circular ring-shaped gasket 3 or the plate-shaped washer 4 is in direct contact with the surface of the bonesetting plate, and then compression fixation is performed by passing through the through holes of the circular ring-shaped gasket 3 or the plate-shaped washer 4 through bolts; when each auxiliary component 2 is the square cushion block 5, the surface of the bonesetting plate is provided with grooves, the surface of each square cushion block 5 is provided with a pin or protrusion, the pins or protrusions of the square cushion blocks 5 are aligned with the grooves in the surface of the bonesetting plate for matching, so that the square cushion blocks 5 are fixedly mounted on the bonesetting plate, the two connection modes can be flexibly selected for different sites and different fracture environments, achieving the optimal bone repair effect, after mounting the bone repair instrument 1 and the auxiliary components 2, the surfaces of the auxiliary components 2 are in direct contact with the repaired bone surface, the surface of the bone repair instrument 1 does not come into contact with the repaired bone surface, and a clearance distance less than or equal to 5 mm is formed, during stepwise degradation of the auxiliary components 2, a fracture surface at a fixation site gradually heals, and substances produced by the degradation are absorbed, at the same time, the volumes of the auxiliary components 2 will gradually decrease due to degradation, such that pressure at the fixation site gradually decreases, the blood supply at a compressed part can be gradually restored to ensure nutrient supply and metabolic circulation at a fracture site, and in addition, a mechanical load is directly transferred to the auxiliary components 2 through the repaired bone surface, and the reaction force of the auxiliary components 2 will well stimulate the functional differentiation of bone cells on the surface which is in contact with the auxiliary components 2 due to the closer elastic moduli of the auxiliary components 2 and the repaired bone, making bone healing more desirable.

In this embodiment, the following specific embodiments are included:
specific embodiment 4-1: provided is a use method of degradable metal blocks combined with a universal bonesetting plate formed by pin fitting, wherein the universal bonesetting plate is a commercially available Ti6Al7Nb bonesetting plate, a structure can be shown in FIGS. 1-3, cushion blocks in the figures are pure magnesium cushion blocks, and a new bone fixation system is formed by combining protrusions on the surfaces of the pure magnesium blocks with recesses in the surface of the Ti6Al7Nb bonesetting plate. During fixation, the surfaces of the pure magnesium blocks are in direct contact with the surface of human bone, while the Ti6Al7Nb bonesetting plate is not in contact with the bone surface, forming a clearance distance less than or equal to 5 mm. During the gradual degradation of the pure magnesium blocks, a fracture surface at a fixation site gradually heals, and magnesium ions generated by the degradation are absorbed. At the same time, the volumes of the magnesium blocks are gradually reduced so that pressure at the fixation site is gradually reduced, and the blood supply at a compressed site can be gradually restored to ensure nutrient supply and metabolic circulation at a fracture site. In addition, a mechanical load is directly transferred to the magnesium blocks through the repaired bone surface, and due to the closer elastic moduli of the magnesium blocks and the repaired bone, the reaction force of the magnesium blocks will well stimulate the functional differentiation of bone cells on the surface which is in contact with the magnesium blocks, making bone healing more desirable.

Embodiment 4-2: provided is a use method of degradable metal blocks combined with a universal bonesetting plate formed by pin fitting, wherein a bone repair instrument 1 is a universal bonesetting plate which is a commercially available stainless steel bonesetting plate, a structure can be shown in FIGS. 1-3, cushion blocks in the figures are pure magnesium cushion blocks, and a new bone fixation system is formed by combining protrusions on the surfaces of the pure magnesium blocks with recesses in the surface of the stainless steel bonesetting plate. During fixation, the surfaces of the pure magnesium blocks are in direct contact with the surface of human bone, while the stainless steel bonesetting plate is not in contact with the bone surface, forming a clearance distance less than or equal to 5 mm. During the gradual degradation of the pure magnesium blocks, a fracture surface at a fixation site gradually heals, and magnesium ions generated by the degradation are absorbed. At the same time, the volumes of the magnesium blocks are gradually reduced so that pressure at the fixation site is gradually reduced, and the blood supply at a compressed site can be gradually restored to ensure nutrient supply and metabolic circulation at a fracture site. In addition, a mechanical load is directly transferred to the magnesium blocks through the repaired bone surface, and due to the closer elastic moduli of the magnesium blocks and the repaired bone, the reaction force of the magnesium blocks will well stimulate the functional differentiation of bone cells on the surface which is in contact with the magnesium blocks, making bone healing more desirable.

Embodiment 4-3: provided is a use method of degradable metal blocks combined with a universal bonesetting plate formed by pin fitting, wherein a bone repair instrument 1 is a universal bonesetting plate which is a commercially available stainless steel bonesetting plate, a structure can be shown in FIGS. 1-3, and cushion blocks in the figures are WE43 magnesium alloy cushion blocks, and a novel bone fixation system is formed by combining protrusions on the surfaces of the WE43 magnesium alloy cushion blocks with recesses in the surface of the stainless steel bonesetting plate. During fixation, the surfaces of the WE43 alloy blocks are in direct contact with the surface of human bone, while the stainless steel bonesetting plate is not in contact with the bone surface, forming a clearance distance less than or equal to 5 mm. During the gradual degradation of the WE43 alloy blocks, a fracture surface at a fixation site gradually heals, and magnesium ions generated by the degradation are absorbed. At the same time, the volumes of the WE43 magnesium alloy blocks are gradually reduced so that pressure at the fixation site is gradually reduced, and the blood supply at a compressed site can be gradually restored to ensure nutrient supply and metabolic circulation at a fracture site. In addition, a mechanical load is directly transferred to the WE43 magnesium alloy blocks through the repaired bone surface, and due to the closer elastic moduli of the WE43 magnesium alloy blocks and the repaired bone, the reaction force of the WE43 alloy blocks will well stimulate the functional differentiation of bone cells on the surface which is in contact with the WE43 alloy blocks, making bone healing more desirable.

Embodiment 4-4: provided is a use method of degradable metal blocks combined with a universal bonesetting plate formed by pin fitting, wherein a bone repair instrument 1 is a universal bonesetting plate which is a commercially available stainless steel bonesetting plate, a structure can be shown in FIGS. 1-3, cushion blocks 5 in the figures are Zn-1Mg alloy cushion blocks, and a new bone fixation system is formed by combining protrusions on the surfaces of the Zn-1Mg alloy blocks and recesses in the surface of the stainless steel bonesetting plate. During fixation, the surfaces of the Zn-1Mg alloy blocks are in direct contact with the surface of human bone, while the stainless steel bonesetting plate is not in contact with the bone surface, forming a clearance distance less than or equal to 5 mm. During the gradual degradation of the Zn-1Mg alloy blocks, a fracture surface at a fixation site gradually heals, and zinc ions, and magnesium ions generated by the degradation are absorbed. At the same time, the volumes of the Zn-1Mg alloy blocks are gradually reduced so that pressure at the fixation site is gradually reduced, and the blood supply at a compressed site can be gradually restored to ensure nutrient supply and metabolic circulation at a fracture site. In addition, a mechanical load is directly transferred to the Zn-1Mg alloy blocks through the repaired bone surface, and due to the closer elastic moduli of the Zn-1Mg alloy blocks and the repaired bone, the reaction force of the Zn-1Mg alloy blocks will well stimulate the functional differentiation of bone cells on the surface which is in contact with the Zn-1Mg alloy blocks, making bone healing more desirable.

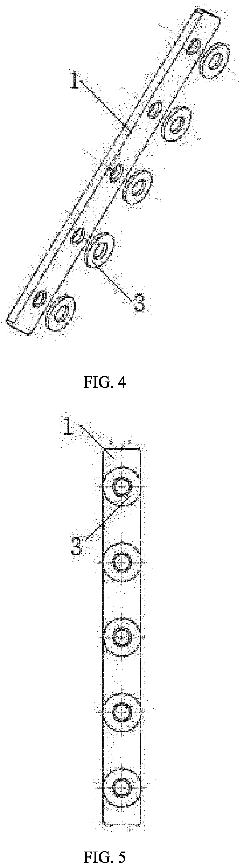

Embodiment 4-5: provided is a use method of degradable metal gaskets combined with a universal bonesetting plate formed by direct contact, wherein a bone repair instrument 1 is a universal bonesetting plate which is a commercially available stainless steel bonesetting plate, a structure can be shown in FIGS. 4-6, the universal bonesetting plate is a commercially available Ti6Al4V bonesetting plate, cushion blocks in the figures are Zn-1Mg alloy gaskets, and the Zn-1Mg alloy gaskets are in direct contact with the surface of the Ti6A14V bonesetting plate to form a new bone fixation system. During fixation, the Zn-1Mg alloy gaskets are in direct contact with the surface of human bone, while the Ti6A14V bonesetting plate is not in contact with the bone surface, forming a certain clearance distance. During the gradual degradation of the Zn-1Mg alloy gaskets, a fracture surface at a fixation site gradually heals, and zinc ions, and magnesium ions generated by the degradation are absorbed. At the same time, the volumes of the Zn-1Mg alloy gaskets are gradually reduced so that pressure at the fixation site is gradually reduced, and the blood supply at a compressed site can be gradually restored to ensure nutrient supply and metabolic circulation at a fracture site. In addition, a mechanical load is directly transferred to the Zn-1Mg alloy gaskets through the repaired bone surface, and due to the closer elastic moduli of the Zn-1Mg alloy gaskets and the repaired bone, the reaction force of the gaskets will well stimulate the functional differentiation of bone cells on the surface which is in contact with the gaskets, making bone healing more desirable.

Embodiment 4-6: provided is a use method of degradable metal gaskets combined with a universal bonesetting plate formed by direct contact, wherein a bone repair instrument 1 is a universal bonesetting plate which is a commercially available stainless steel bonesetting plate, a structure can be shown in FIGS. 4-6, the universal bonesetting plate is a commercially available Ti6Al4V bonesetting plate, cushion blocks in the figures are HA/Mg-Zn-Ca composite gaskets, and the HA/Mg-Zn-Ca composite gaskets are in direct contact with the surface of the Ti6A14V bonesetting plate to form a new bone fixation system. During fixation, the HA/Mg-Zn-Ca composite gaskets are in direct contact with the surface of human bone, while the Ti6A14V bonesetting plate is not in contact with the bone surface, forming a certain clearance distance. During the gradual degradation of the HA/Mg-Zn-Ca composite gaskets, a fracture surface at a fixation site gradually heals, and zinc ions, magnesium, calcium ions and HA generated by the degradation are absorbed. At the same time, the volumes of the HA/Mg-Zn-Ca composite gaskets are gradually reduced so that pressure at the fixation site is gradually reduced, and the blood supply at a compressed site can be gradually restored to ensure nutrient supply and metabolic circulation at a fracture site. In addition, a mechanical load is directly transferred to the HA/Mg-Zn-Ca composite gaskets through the repaired bone surface, and due to the closer elastic moduli of the HA/Mg-Zn-Ca composite gaskets and the repaired bone, the reaction force of the gaskets will well stimulate the functional differentiation of bone cells on the surface which is in contact with the gaskets, making bone healing more desirable.

Embodiment 4-7: provided is a use method of degradable metal gaskets combined with a universal bonesetting plate formed by direct contact, wherein a bone repair instrument 1 is a universal bonesetting plate which is a commercially available stainless steel bonesetting plate, a structure can be shown in FIGS. 4-6, the universal bonesetting plate is a commercially available stainless steel bonesetting plate, cushion blocks in the figures are $\beta$-TCP/Zn-Mg composite gaskets, and the $\beta$-TCP/Zn-Mg composite gaskets are in direct contact with the surface of the stainless steel bonesetting plate to form a new bone fixation system. During fixation, the $\beta$-TCP/Zn-Mg composite gaskets are in direct contact with the surface of human bone, while the stainless steel bonesetting plate is not in contact with the bone surface, forming a certain clearance distance. During the gradual degradation of the $\beta$-TCP/Zn-Mg composite gaskets, a fracture surface at a fixation site gradually heals, and zinc ions, magnesium ions, $\beta$-TCP, etc. generated by the degradation are absorbed. At the same time, the volumes of the $\beta$-TCP/Zn-Mg composite gaskets are gradually reduced so that pressure at the fixation site is gradually reduced, and the blood supply at a compressed site can be gradually restored to ensure nutrient supply and metabolic circulation at a fracture site. In addition, a mechanical load is directly transferred to the $\beta$-TCP/Zn-Mg composite gaskets through the repaired bone surface, and due to the closer elastic moduli of the $\beta$-TCP/Zn-Mg composite gaskets and the repaired bone, the reaction force of the gaskets will well stimulate the functional differentiation of bone cells on the surface which is in contact with the gaskets, making bone healing more desirable.

Embodiment 4-8: provided is a use method of degradable metal plates combined with a universal bonesetting plate formed by direct contact, wherein a bone repair instrument 1 is a universal bonesetting plate which is a commercially available stainless steel bonesetting plate, a structure can be shown in FIGS. 4-6, the universal bonesetting plate is a commercially available pure titanium bonesetting plate, plates in the figures are $\beta$-TCP/Zn-Mg composite plates, and the $\beta$-TCP/Zn-Mg composite plates are in direct contact with the surface of the titanium alloy bonesetting plate to form a new bone fixation system. During fixation, the $\beta$-TCP/Zn-Mg composite plates are in direct contact with the surface of human bone, while the pure titanium bonesetting plate is not in contact with the bone surface, forming a certain clearance distance. During the gradual degradation of the $\beta$-TCP/Zn-Mg composite plates, a fracture surface at a fixation site gradually heals, and zinc ions, magnesium ions, $\beta$-TCP, etc. generated by the degradation are absorbed. At the same time, the volumes of the $\beta$-TCP/Zn-Mg composite plates are gradually reduced so that pressure at the fixation site is gradually reduced, and the blood supply at a compressed site can be gradually restored to ensure nutrient supply and metabolic circulation at a fracture site. In addition, a mechanical load is directly transferred to the β-TCP/Zn-Mg composite plates through the repaired bone surface, and due to the closer elastic moduli of the β-TCP/Zn-Mg composite plates and the repaired bone, the reaction force of the composite plates will well stimulate the functional differentiation of bone cells on the surface which is in contact with the composite plates, making bone healing more desirable.

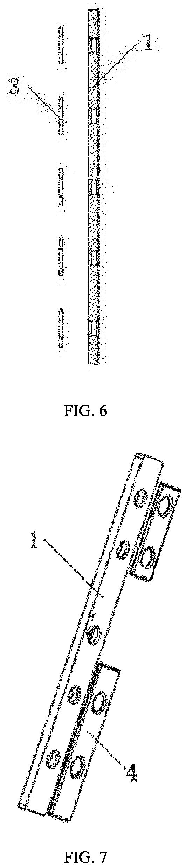

Embodiment 4-9: provided is a use method of degradable metal plates combined with a universal bonesetting plate formed by direct contact, wherein a bone repair instrument 1 is a universal bonesetting plate which is a commercially available stainless steel bonesetting plate, a structure can be shown in FIGS. 7-9, the universal bonesetting plate is a commercially available stainless steel bonesetting plate, plates in the figures are Mg-Y alloy plates, and the Mg-Y alloy plates are in direct contact with the surface of the stainless steel bonesetting plate to form a new bone fixation system. During fixation, the Mg-Y alloy plates are in direct contact with the surface of human bone, while the stainless steel bonesetting plate is not in contact with the bone surface, forming a certain clearance distance. During the gradual degradation of the Mg-Y alloy plates, a fracture surface at a fixation site gradually heals, and magnesium ions generated by the degradation are absorbed. At the same time, the volumes of the Mg-Y alloy plates are gradually reduced so that pressure at the fixation site is gradually reduced, and the blood supply at a compressed site can be gradually restored to ensure nutrient supply and metabolic circulation at a fracture site. In addition, a mechanical load is directly transferred to the Mg-Y alloy plates through the repaired bone surface, and due to the closer elastic moduli of the Mg-Y alloy plates and the repaired bone, the reaction force of the alloy plates will well stimulate the functional differentiation of bone cells on the surface which is in contact with the alloy plates, making bone healing more desirable.

Embodiment 4-10: provided is a use method of degradable metal plates combined with a customized personalized prosthesis formed by direct contact, wherein a substrate of the customized personalized prosthesis is pure titanium. Pure magnesium washers are placed in a direct contact manner between the customized prosthesis and the fixed repaired bone surface to form a new bone fixation system. During fixation, the pure magnesium washers are in direct contact with the surface of human bone, while the customized individualized prosthesis is not in contact with the bone surface, forming a certain clearance distance. During the gradual degradation of the pure magnesium washers, a bone defect site at a fixation site gradually heals, and magnesium ions generated by the degradation are absorbed. At the same time, the volumes of the pure magnesium washers are gradually reduced so that pressure at the fixation site is gradually reduced, and the blood supply at a compressed site can be gradually restored to ensure nutrient supply and metabolic circulation at a fracture site. In addition, a mechanical load is directly transferred to the pure magnesium washers through the repaired bone surface, and due to the closer elastic moduli of the pure magnesium washers and the repaired bone, the reaction force of the pure magnesium washers will well stimulate the functional differentiation of bone cells on the surface which is in contact with the pure magnesium washers, making bone healing more desirable.

Embodiment 4-11: provided is a use method of degradable metal blocks combined with a universal bonesetting plate formed by pin fitting, wherein a bone repair instrument 1 is a universal bonesetting plate which is a commercially available stainless steel bonesetting plate, a structure can be shown in FIGS. 1-3, the universal bonesetting plate is a commercially available Ti6Al7Nb bonesetting plate, cushion blocks in the figures are pure iron cushion blocks, and a new bone fixation system is formed by combining protrusions on the surfaces of the pure iron blocks with recesses in the surface of the Ti6Al7Nb bonesetting plate. During fixation, the surfaces of the pure iron blocks are in direct contact with the surface of human bone, while the Ti6Al7Nb bonesetting plate is not in contact with the bone surface, forming a clearance distance less than or equal to 5 mm. During the gradual degradation of the pure iron blocks, a fracture surface at a fixation site gradually heals, and iron ions generated by the degradation are absorbed. At the same time, the volumes of the pure iron blocks are gradually reduced so that pressure at the fixation site is gradually reduced, and the blood supply at a compressed site can be gradually restored to ensure nutrient supply and metabolic circulation at a fracture site. In addition, a mechanical load is directly transferred to the pure iron blocks through the repaired bone surface, and due to the closer elastic moduli of the pure iron blocks and the repaired bone, the reaction force of the pure iron blocks will well stimulate the functional differentiation of bone cells on the surface which is in contact with the pure iron blocks, making bone healing more desirable.

Embodiment 4-12: provided is a use method of degradable metal gaskets combined with a universal bonesetting plate formed by direct contact, wherein a bone repair instrument 1 is a universal bonesetting plate which is a commercially available stainless steel bonesetting plate, a structure can be shown in FIGS. 4-6, the universal bonesetting plate is a commercially available Ti6Al4V bonesetting plate, cushion blocks in the figures are Fe-5Mn alloy gaskets, and the Fe-5Mn alloy gaskets are in direct contact with the surface of the Ti6A14V bonesetting plate to form a new bone fixation system. During fixation, the Fe-5Mn alloy gaskets are in direct contact with the surface of human bone, while the Ti6A14V bonesetting plate is not in contact with the bone surface, forming a certain clearance distance. During the gradual degradation of the Fe-5Mn alloy gaskets, a fracture surface at a fixation site gradually heals, and iron ions, and manganese ions generated by the degradation are absorbed. At the same time, the volumes of the Fe-5Mn alloy gaskets are gradually reduced so that pressure at the fixation site is gradually reduced, and the blood supply at a compressed site can be gradually restored to ensure nutrient supply and metabolic circulation at a fracture site. In addition, a mechanical load is directly transferred to the Fe-5Mn alloy gaskets through the repaired bone surface, and due to the closer elastic moduli of the Fe-5Mn alloy gaskets and the repaired bone, the reaction force of the Fe-5Mn alloy gaskets will well stimulate the functional differentiation of bone cells on the surface which is in contact with the Fe-5Mn alloy gaskets, making bone healing more desirable.

Embodiment 4-13: provided is a use method of degradable metal plates combined with a universal bonesetting plate formed by direct contact, wherein a bone repair instrument 1 is a universal bonesetting plate which is a commercially available stainless steel bonesetting plate, a structure can be shown in FIGS. 7-9, the universal bonesetting plate is a commercially available pure titanium bonesetting plate, plates in the figures are CNT/Fe composite plates, and the CNT/Fe composite plates are in direct contact with the surface of the stainless steel bonesetting plate to form a new bone fixation system. During fixation, the CNT/Fe composite plates are in direct contact with the surface of human bone, while the stainless steel bonesetting plate is not in contact with the bone surface, forming a certain clearance distance. During the gradual degradation of the CNT/Fe composite plates, a fracture surface at a fixation site gradually heals, and iron ions generated by the degradation are absorbed. At the same time, the volumes of the CNT/Fe composite plates are gradually reduced so that pressure at the fixation site is gradually reduced, and the blood supply at a compressed site can be gradually restored to ensure nutrient supply and metabolic circulation at a fracture site. In addition, a mechanical load is directly transferred to the CNT/Fe composite plates through the repaired bone surface, and due to the closer elastic moduli of the CNT/Fe composite plates and the repaired bone, the reaction force of the composite plates will well stimulate the functional differentiation of bone cells on the surface which is in contact with the composite plates, making bone healing more desirable.

Embodiment 4-14: provided is a use method of degradable metal blocks combined with a universal bonesetting plate formed by pin fitting, wherein a bone repair instrument 1 is a universal bonesetting plate which is a commercially available stainless steel bonesetting plate, a structure can be shown in FIGS. 1-3, the universal bonesetting plate is a commercially available stainless steel bonesetting plate, cushion blocks in the figures are anodized pure magnesium cushion blocks, and a new bone fixation system is formed by combining protrusions on the surfaces of the pure magnesium blocks with recesses in the surface of the stainless steel bonesetting plate. During fixation, the surfaces of the anodized pure magnesium blocks are in direct contact with the surface of human bone, while the stainless steel bonesetting plate is not in contact with the bone surface, forming a clearance distance less than or equal to 5 mm. The corrosion resistance of the anodized pure magnesium cushion blocks becomes stronger, which is more conducive to rigid mechanical fixation in the early stage of healing at a fracture site. As a fracture surface gradually heals, pure magnesium begins to gradually degrade, and magnesium ions produced by the degradation are absorbed by the body. At the same time, the volumes of the magnesium blocks are gradually reduced so that pressure at the fixation site is gradually reduced, and the blood supply at a compressed site can be gradually restored to ensure nutrient supply and metabolic circulation at a fracture site. In addition, a mechanical load is directly transferred to the magnesium blocks through the repaired bone surface, and due to the closer elastic moduli of the magnesium blocks and the repaired bone, the reaction force of the magnesium blocks will well stimulate the functional differentiation of bone cells on the surface which is in contact with the magnesium blocks, making bone healing more desirable.

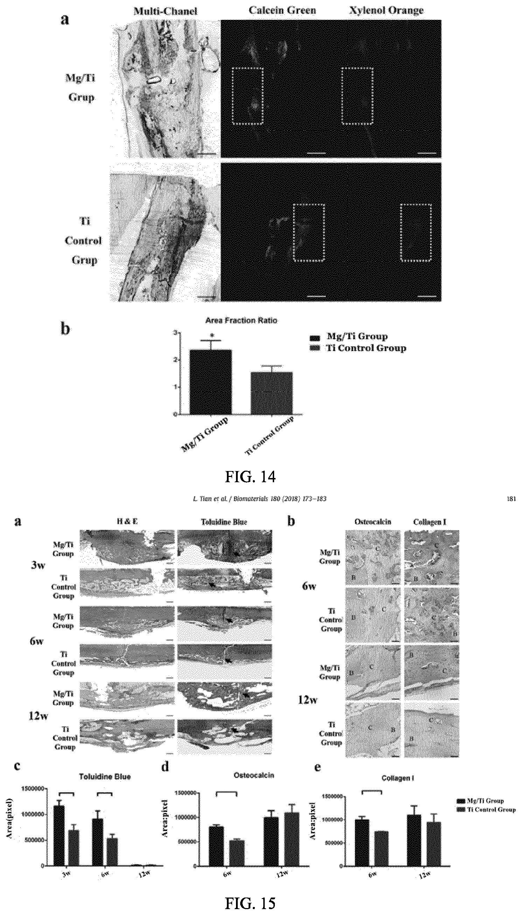

Embodiment 4-15: provided is a use method of degradable metal gaskets combined with a universal bonesetting plate formed by direct contact, wherein a bone repair instrument 1 is a universal bonesetting plate which is a commercially available stainless steel bonesetting plate, a structure can be shown in FIGS. 4-6, the universal bonesetting plate is a commercially available Ti6A14V bonesetting plate, as shown in FIG. 15, cushion blocks in the figure are Zn-1Mg alloy gaskets coated with an HA coating prepared by chemical precipitation, and the Zn-1Mg alloy gaskets coated with the HA coating are in direct contact with the surface of the titanium alloy bonesetting plate to form a new bone fixation system. During fixation, the HA coatings on the surfaces of the Zn-1Mg alloy gaskets are in direct contact with the surface of human bone, and the presence of HA will induce differentiation of osteoblasts on the surface of damaged bone to be repaired, thereby having good surface activity. The Ti6A14V bonesetting plate is not in contact with the bone surface, forming a certain clearance distance. During the gradual degradation of the Zn-1Mg alloy gaskets, a fracture surface at a fixation site gradually heals, and zinc ions, and magnesium ions generated by the degradation are absorbed. At the same time, the volumes of the Zn-1Mg alloy gaskets are gradually reduced so that pressure at the fixation site is gradually reduced, and the blood supply at a compressed site can be gradually restored to ensure nutrient supply and metabolic circulation at a fracture site. In addition, a mechanical load is directly transferred to the Zn-1Mg alloy gaskets through the repaired bone surface, and due to the closer elastic moduli of the Zn-1Mg alloy gaskets and the repaired bone, the reaction force of the gaskets will well stimulate the functional differentiation of bone cells on the surface which is in contact with the gaskets, making bone healing more desirable.

Embodiment 4-16: provided is a use method of degradable metal plates combined with a universal bonesetting plate formed by pin fitting, wherein a bone repair instrument 1 is a universal bonesetting plate which is a commercially available stainless steel bonesetting plate, a structure can be shown in FIGS. 7-9, the universal bonesetting plate is a commercially available stainless steel bonesetting plate, plates in the figures are WE43 magnesium alloy plates after micro-arc oxidation, and a new fixation system is formed by direct contact of the WE43 magnesium alloy plates with the surface of the stainless steel bonesetting plate. During fixation, the surfaces of the WE43 alloy plates after micro-arc oxidation are in direct contact with the surface of human bone, while the stainless steel bonesetting plate is not in contact with the bone surface, forming a certain clearance distance. A rate of degradation of the WE43 alloy plates after micro-arc oxidation is slower during the repair process compared with pure magnesium plates, which is beneficial to the early rigid mechanical fixation at a fracture site. During the gradual degradation of the alloy in a later stage, a fracture surface at a fixation site gradually heals, and magnesium ions generated by the degradation are absorbed. At the same time, the volumes of the WE43 magnesium alloy plates are gradually reduced so that pressure at the fixation site is gradually reduced, and the blood supply at a compressed site can be gradually restored to ensure nutrient supply and metabolic circulation at the fracture site. In addition, a mechanical load is directly transferred to the WE43 magnesium alloy plates through the repaired bone surface, and due to the closer elastic moduli of the WE43 magnesium alloy plates and the repaired bone, the reaction force of the WE43 alloy plates will well stimulate the functional differentiation of bone cells on the surface which is in contact with the WE43 alloy plates, making bone healing more desirable.

Embodiment 4-17: provided is a use method of degradable metal blocks combined with a universal bonesetting plate formed by pin fitting, wherein a bone repair instrument 1 is a universal bonesetting plate which is a commercially available stainless steel bonesetting plate, a structure can be shown in FIGS. 1-3, the universal bonesetting plate is a commercially available stainless steel bonesetting plate, cushion blocks in the figures are anodized pure magnesium cushion blocks, and a new bone fixation system is formed by combining protrusions on the surfaces of the pure magnesium blocks with recesses in the surface of the stainless steel bonesetting plate. During fixation, the surfaces of the anodized pure magnesium blocks are in direct contact with the surface of human bone, while the stainless steel bonesetting plate is not in contact with the bone surface, forming a clearance distance less than or equal to 5 mm. The corrosion resistance of the anodized pure magnesium cushion blocks becomes stronger, which is more conducive to rigid mechanical fixation in the early stage of healing at a fracture site. As a fracture surface gradually heals, pure magnesium begins to gradually degrade, and magnesium ions produced by the degradation are absorbed by the body. At the same time, the volumes of the magnesium blocks are gradually reduced so that pressure at the fixation site is gradually reduced, and the blood supply at a compressed site can be gradually restored to ensure nutrient supply and metabolic circulation at a fracture site. In addition, a mechanical load is directly transferred to the magnesium blocks through the repaired bone surface, and due to the closer elastic moduli of the magnesium blocks and the repaired bone, the reaction force of the magnesium blocks will well stimulate the functional differentiation of bone cells on the surface which is in contact with the magnesium blocks, making bone healing more desirable.

Embodiment 4-18: provided is a use method of degradable polymer plates combined with a universal bonesetting plate formed by pin fitting, wherein a bone repair instrument 1 is a universal bonesetting plate which is a commercially available stainless steel bonesetting plate, a structure can be shown in FIGS. 4-6, the universal bonesetting plate is a commercially available stainless steel bonesetting plate, plates in the figures are polylactic acid (PLA) plates, and a new fixation system is formed by direct contact of the polylactic acid (PLA) plates with the surface of the stainless steel bonesetting plate. During fixation, the surfaces of the polylactic acid (PLA) plates are in direct contact with the surface of human bone, while the stainless steel bonesetting plate is not in contact with the bone surface, forming a certain clearance distance. The polylactic acid (PLA) plates gradually degrade during the repair process, a fracture surface at a fixation site gradually heals, and lactic acid molecules produced by the degradation are absorbed. At the same time, the volumes of the polylactic acid (PLA) plates are gradually reduced so that pressure at the fixation site is gradually reduced, and the blood supply at a compressed site can be gradually restored to ensure nutrient supply and metabolic circulation at a fracture site. In addition, a mechanical load is directly transferred to the polylactic acid (PLA) plates through the repaired bone surface, and due to the closer elastic moduli of the polylactic acid (PLA) plates and the repaired bone, the reaction force of the polylactic acid (PLA) plates will well stimulate the functional differentiation of bone cells on the surface which is in contact with the polylactic acid (PLA) plates, making bone healing more desirable.

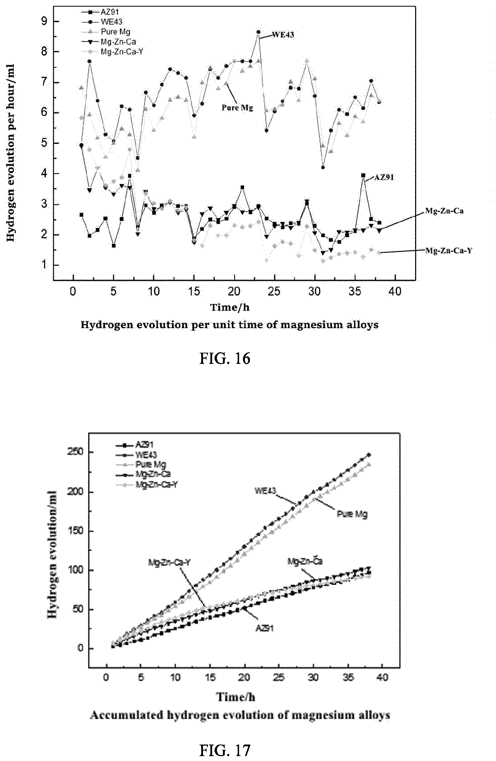

Embodiment 4-19: provided is a use method of degradable polymer blocks combined with a universal bonesetting plate formed by pin fitting, wherein a bone repair instrument 1 is a universal bonesetting plate which is a commercially available stainless steel bonesetting plate, a structure can be shown in FIGS. 1-3, the universal bonesetting plate is a commercially available stainless steel bonesetting plate, as shown in FIG. 17, cushion blocks in the figure are polycaprolactone (PCL) cushion blocks, and a new bone fixation system is formed by combining protrusions on the surfaces of the polycaprolactone (PCL) blocks with recesses in the surface of the stainless steel bonesetting plate. During fixation, the surfaces of the polycaprolactone (PCL) blocks are in direct contact with the surface of human bone, while the stainless steel bonesetting plate is not in contact with the bone surface, forming a clearance distance less than or equal to 5 mm. As a fracture surface gradually heals, polycaprolactone (PCL) begins to gradually degrade, and monomers produced by the degradation are absorbed by the body. At the same time, the volumes of the polycaprolactone (PCL) blocks are gradually reduced so that pressure at the fixation site is gradually reduced, and the blood supply at a compressed site can be gradually restored to ensure nutrient supply and metabolic circulation at a fracture site. In addition, a mechanical load is directly transferred to the polycaprolactone (PCL) blocks through the repaired bone surface, and due to the closer elastic moduli of the polycaprolactone (PCL) blocks and the repaired bone, the reaction force of the polycaprolactone (PCL) blocks will well stimulate the functional differentiation of bone cells on the surface which is in contact with the polycaprolactone (PCL) blocks, making bone healing more desirable.

One experiment of the present invention was as follows:

structure: high purity magnesium screw/titanium alloy bonesetting plate, and titanium screw/titanium alloy bonesetting plate;

magnesium screw treatment: in order to avoid electrochemical corrosion caused by direct contact of a magnesium screw with a titanium plate, a magnesium thread head, and a part of threads next to the thread head were coated with a layer of polylactic acid; and

experiment: Z-shaped incision osteotomy was performed in a middle segment of a rabbit tibia, and fractured tibia was fixed with a titanium plate, a titanium screw and one coated magnesium screw in the middle, and a control group adopted titanium screws. The magnesium screw was designed to be placed near a fracture site to fix bone fragments.

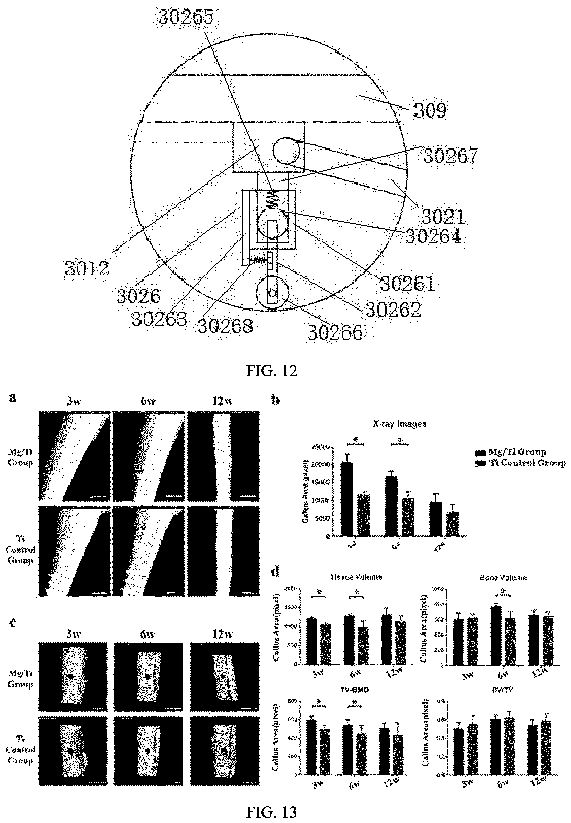

Experimental results: 1. both the Mg/Ti group and the Ti control group had callus formation around the fracture site, and the callus tissue area, the callus volume, and the bone density in the Mg/Ti group were higher than those in the Ti control group at 3 and 6 weeks after surgery, referring to FIG. 13;

2. fluorescence labeled non-decalcified sections showed that there were mineral deposits at the fracture site at 6 weeks after surgery, exactly in the direction of a fracture gap, and quantitative analysis showed that a bone formation rate at the fracture site in the Mg/Ti group was about 53.2%, which was significantly higher than that in the Ti control group, referring to FIG. 14;

3. HE staining, toluidine blue staining and type I collagen immunohistochemical staining showed that there was a larger callus tissue area in the Mg/Ti composite group, referring to FIG. 15; and

4. there was no subcutaneous hydrogen accumulation at a surgical site.

Another experiment of the present invention was as follows:
according to the design purpose of this experiment, the used metal raw materials included: Pure Mg, WE43, Mg-Zn-Ca, Mg-Zn-Ca-Y, AZ91, Pure Zn, Zn-1Mg and Ti6A14V.

Items measured during the experiment:

① gas evolution after a soaked sample was in contact with a simulated body fluid was mainly recorded, and the rate and location of bubble evolution, and changes in the surface condition of the sample (product formation, change in smoothness, etc.) were observed. Hydrogen evolution was determined once every hour.
(2) pH measurement: measurement was performed once every two hours for magnesium alloys; and measurement was performed once a day for zinc alloys.
③ The simulated body fluid was sampled to determine the concentration of key ions in the solution.
④ Observation and compositional determination (SEM, XRD, and EDS) of corrosion products on the surface of the sample.
⑤ Weight loss statistics: after removal of the corrosion products, the surface morphology of the sample was observed, mass loss was measured, and a weight loss rate was calculated.

Conclusion: from hydrogen evolution per hour and accumulated hydrogen evolution, it can be seen that the corrosion rates of WE43 and pure Mg were the fastest, and the corrosion rates of AZ91, Mg-Zn-Ca and Mg-Zn-Ca-Y were comparable, which were lower than those of WE43 and pure Mg, referring to FIGS. 16 and 17;

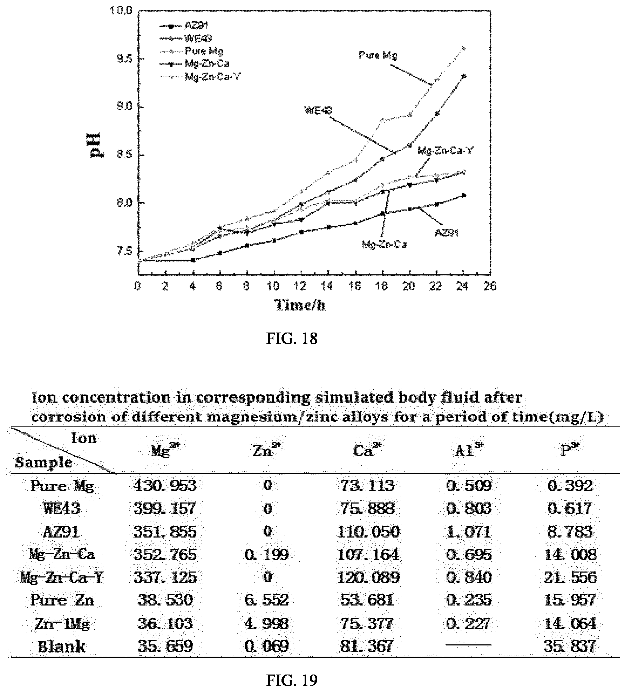

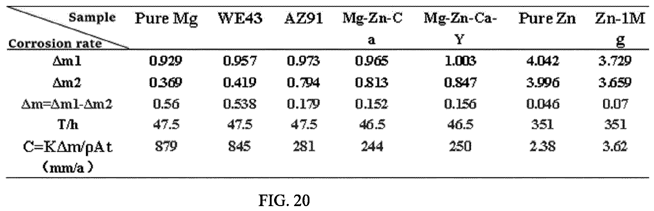

from the change of a pH value, it can be seen that a pH value of WE43 and a pH value of pure Mg increased more rapidly, and the concentration of $OH^-$ ions in the solution increased, indicating a fast hydrogen evolution rate, which was consistent with the results of a hydrogen evolution diagram. Wherein the change in a pH value of AZ91 was minimal, and it was preliminarily determined that AZ91 was more corrosion resistant. FIG. 18 is a pH curve, FIG. 19 shows an ion concentration, and FIG. 20 shows a sample corrosion rate;

1. degradable metals used in the experiments were all as-cast materials, and the corrosion rate was increased compared with corresponding machined materials;

2. a contact corrosion experiment was generally a galvanic reaction, which included a galvanic reaction formed by contact corrosion of a magnesium alloy, a zinc alloy and a titanium alloy, and a galvanic reaction formed inside the alloys due to an electric potential difference between different structures inside the alloys;

3. from the longitudinal comparison, the reaction of the magnesium alloy was more vigorous, and the reaction process was accompanied by vigorous gas generation and continuous accumulation of corrosion products. Among magnesium alloys, WE43 and Pure Mg reacted most violently, a alloy matrix will continuously disintegrate with the reaction process, and a large amount of magnesium alloy chips were mixed in corrosion products observed at the bottom of a soaking bottle;

4. the zinc alloy exhibited relatively stable performance in contact corrosion, with corrosion concentrated at certain points and almost no corrosion at other locations, making it an advantageous candidate material; and

5. from the initial experimental results, the contact corrosion of as-cast magnesium alloys was generally fast, and the corrosion process of Zn alloys was relatively stable, the two had their own advantages, and a combination of the two materials was considered the most promising experimental process for achieving AO-BO, i.e., zinc coated magnesium composite washers. Possible implementations are currently: hot dip plating and mechanical compounding.

Embodiment 5, on the basis of any one of Embodiments 1-4

The surface of each auxiliary component is subjected to surface treatment including any one of micro-arc oxidation, chemical deposition, and anodization.

A working principle and the beneficial effects of the above technical solution are as follows: micro-arc oxidation, chemical deposition or anodization is performed on the surfaces of auxiliary components 2, a degradation rate of the auxiliary components 2 can be reduced, and meanwhile, by performing chemical deposition on the surfaces of the auxiliary components 2, the biocompatibility of the surfaces of the auxiliary components 2 can be increased, which is less likely to cause discomfort for patients and helps to heal a fracture site.

Embodiment 6, on the basis of any one of Embodiments 1-5

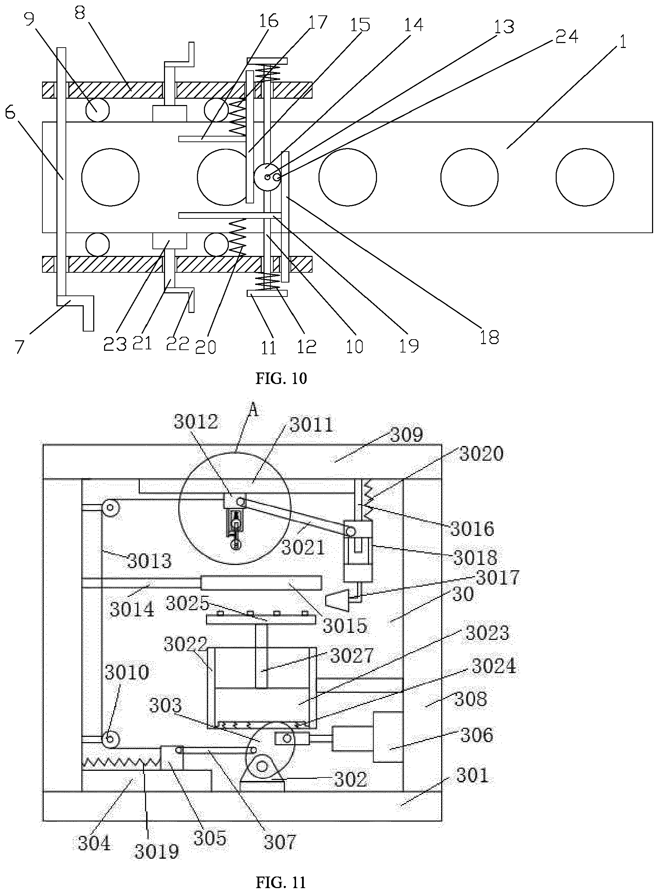

As shown in FIG. 10, the fracture repair device further includes a mounting device, wherein the mounting device includes:

a first screw 6, wherein the first screw 6 is disposed above the bone repair instrument 1, the first screw 6 is a double-headed screw, two ends of the first screw 6 are oppositely threaded, and one end of the first screw 6 is provided with a first rocker 7;
moving plates 8, wherein the two moving plates 8 are symmetrically disposed on two sides of the bone repair instrument 1, the two moving plates 8 are respectively threadedly connected with two ends of the first screw 6, and a plurality of rollers 9 are arranged on one side, facing the bone repair instrument 1, of each moving plate 8;
a guide plate 10, wherein the guide plate 10 is disposed at one ends, away from the first screw 6, of the moving plates 8, two ends of the guide plate 10 respectively penetrate through the moving plates 8 on both sides, and are slidably connected with the moving plates 8, two ends of the guide plate 10 each are provided with a baffle 11, a first spring 12 is disposed between each baffle 11 and the corresponding moving plate 8, one end of each first spring 12 is fixedly connected with a side wall of the corresponding moving plate 8, and the other end of each first spring 12 is fixedly connected with a side wall of the corresponding baffle 11;
a first rotary shaft 13, wherein the first rotary shaft 13 is disposed above the guide plate 10, the first rotary shaft 13 is perpendicular to the bone repair instrument 1, one end of the first rotary shaft 13 is rotatably connected to an upper surface of the guide plate 10, the other end of the first rotary shaft 13 is provided with a gear 14, an upper surface of the gear 14 is provided with a fixed column 24, a first rack 15 is arranged on a left side of the gear 14, the first rack 15 is meshed with the gear 14, one end, away from the gear 14, of the first rack 15 penetrates through the moving plate 8 at an upper part, and is slidably connected with the moving plate 8, one side, away from the guide plate 10, of the first rack 15 is provided with a first clamp plate 16, a second spring 17 is arranged between the first clamp plate 16 and the moving plate 8, one end of the second spring 17 is fixedly connected to a side wall of the first clamp plate 16, the other end of the second spring 17 is fixedly connected with a side wall of the moving plate 8, a second rack 18 is arranged on a right side of the gear 14, the second rack 18 is meshed with the gear 14, one end, away from the gear 14, of the second rack 18 penetrates through the moving plate 8 at a lower part, and is slidably connected with the moving plate 8, one side, close to the guide plate 10, of the second rack 18 is provided with a second clamp plate 19, a third spring 20 is arranged between the second clamp plate 19 and the moving plate 8, one end of the third spring 20 is fixedly connected with a side wall of the moving plate 8, and the other end of the third spring 20 is fixedly connected with a side wall of the second clamp plate 19; and

second screws 21, wherein the two second screws 21 are symmetrically disposed on both sides of the bone repair instrument 1, each second screw 21 is threadedly connected with the middle of the corresponding moving plate 8, one end of each second screw 21 is provided with a second rocker 22, the other end of each second screw 21 is provided with a pressing plate 23, and the pressing plates 23 are in contact with a side wall of the bone repair instrument 1.

A working principle and the beneficial effects of the above technical solution are as follows: since the auxiliary components 2 may be in various forms of a circular ring-shaped gasket, a plate-shaped washer or a square cushion block, when the auxiliary components 2 are mounted on the bone repair instrument 1, if the auxiliary components 2 are not aligned with the bone repair instrument 1, an offset of the auxiliary components 2 occurs, pressures at mounting sites are different, which will affect fracture healing, the mounting device is thus arranged, first by rotating the first rocker 7, the first rocker 7 drives the first screw 6 to rotate, due to the fact that two ends of the first screw 6 are oppositely threaded, the rotation of the first screw 6 drives the two moving plates 8 to simultaneously move towards the bone repair instrument 1 until the rollers 9 are completely in contact with the moving plates 8, the rotation of the first rocker 7 is stopped, at this time, the mounting device is fixed to the surface of the bone repair instrument 1, then the auxiliary components 2 are taken, the auxiliary components 2 may be circular ring-shaped gaskets, plate-shaped washers or square cushion blocks, the auxiliary components 2 are placed on the surface of the bone repair instrument 1, while the auxiliary components 2 are located between the first clamp plate 16 and the second clamp plate 19, the fixed column 24 is rotated, the fixed column 24 drives the gear 14 to rotate, the gear 14 drives the first gear rack 15 and the second gear rack 18 to move, the first rack 15 and the second rack 18 respectively drive the first clamp plate 16 and the second clamp plate 19 to move towards the auxiliary components 2, a distance between the first clamp plate 16 and the moving plate 8 at the upper part is the same as a distance between the second clamp plate 19 and the moving plate 8 at the lower part, the first clamp plate 16 and the second clamp plate 19 move simultaneously and at the same distance, so that the auxiliary components 2 can be clamped, and the auxiliary components 2 are located at the center in the width direction of the bone repair instrument 1, then the second rockers 22 are rotated, the second rockers 22 drive the second screws 21 to rotate, the second screws 21 drive the pressing plates 23 to move towards the bone repair instrument 1, thereby fixing the mounting device to the bone repair instrument 1, preventing the mounting device from sliding, thereby clamping the auxiliary components 2 with the first clamp plate 16 and the second clamp plate 19, facilitating the mounting of the auxiliary components 2, after the auxiliary components 2 are mounted, the fixed column 24 is loosened, under the action of the second spring 17 and the third spring 20, the first clamp plate 16 and the second clamp plate 19 move away from the auxiliary components 2, respectively, then the second rockers 22 are rotated counterclockwise, so that the pressing plates 23 are away from the bone repair instrument 1, at this time, the mounting device can slide on a side wall of the bone repair instrument 1 by means of the rollers 9, facilitating the mounting of the next auxiliary component 2, by providing the mounting device, the auxiliary components 2 can be clamped by the first clamp plate 16 and the second clamp plate 19 when the auxiliary components 2 are mounted to avoid an offset of the positions of the auxiliary components 2 during mounting, resulting in different contact positions of the surfaces of the auxiliary components 2 with the repaired bone surface, thereby reducing the rate of healing of the fracture site, the auxiliary components 2 can be aligned with the bone repair instrument 1 in mounting positions by the mounting device so that the auxiliary components 2 are in sufficient contact with the repaired bone surface.

Embodiment 7, on the basis of any one of Embodiments 1-6, the fracture repair device further includes:

an image acquisition device, disposed above the bone repair instrument 1, and configured to acquire an image that the auxiliary components 2 are mounted on the bone repair instrument 1, and generate a first image;
a processing device connected to the image acquisition device, wherein the processing device performs processing on the first image to equally divide the first image into a plurality of actual image regions;
a controller connected with the processing device, and configured to compare the first image with a preset standard image (which may refer to an image within a size standard range), and generate a comparison result; and
an alarm electrically connected to the controller; wherein
the controller controls the operation of the alarm based on the comparison result, which includes the following steps of:

step 1: calculating an offset obtained by comparing the first image with a preset standard image by a formula (1):

$$\varphi = \frac{\pi}{\sqrt{e*(1+e)^2}} * \sum_{i=1}^{N}(f_i - g_i)^2 *(f_i + g_i) \quad (1)$$

wherein $\varphi$ is an offset obtained by comparing the first image with a preset standard image, e is a constant, e is 2.72, $\pi$ is a constant, $\pi$ is 3.14, N is the total number of the actual image regions in the first image, $f_i$ is a gray value of an ith actual image region in the first image, and $g_i$ is a gray value of an ith preset standard image region in the preset standard image;

step 2: calculating an actual similarity between the first image and the preset standard image by a formula (2):

$$simY = \varphi * \frac{\sum_{i=1}^{N}(f_i * g_i)}{\sqrt[4]{\sum_{i=1}^{N}f_i^2 * \sum_{i=1}^{N}\left(\frac{g_i}{f_i}\right)^2}} * \frac{1}{N}$$

wherein simY is an actual similarity between the first image and the preset standard image; and step 3: comparing, by the controller, the actual similarity between the first image and the preset standard image with a preset similarity, and when the actual similarity is less than the preset similarity, controlling, by the controller, the alarm to give an alarm.

A working principle and the beneficial effects of the above technical solution are as follows: after the auxiliary components 2 are mounted on the bone repair instrument 1, a mounting image is acquired with the image acquisition device, and a first image is correspondingly generated, then the first image is uploaded to the processing device, the first image is processed by the processing device to equally divide the first image into the plurality of actual image regions, the controller may then compare the first image with the preset standard image, the offset between the first image and the preset standard image can be calculated by the formula (1), according to the offset calculated by the formula (1), taking into account the error during comparison, the reliability of the comparison is improved, a similarity between the first image and the preset standard image is calculated by the formula (2), the preset standard image is an image that the auxiliary components 2 are correctly mounted on the metal repair instrument 1, when the similarity between the first image and the preset standard image is greater than or equal to the preset similarity, it is indicated that the auxiliary components 2 are correctly mounted on the metal repair instrument 1, when the similarity between the first image and the preset standard image is less than the preset similarity, it is indicated that an offset of the mounting positions of the auxiliary components 2 occurs, and at this time, the controller controls the alarm to give an alarm, prompting operators to adjust the positions of the auxiliary components 2.

Embodiment 8, based on any one of Embodiments 1-7, as shown in FIGS. 11-12;

the bone repair instrument 1 includes a bonesetting plate, and the fracture repair device further includes: a pretreatment device 30, wherein the pretreatment device 30 includes: a fixed base 301, wherein left and right sides of an upper end of the fixed base 301 are fixedly connected to a horizontal mounting plate 309 through vertical brackets 308; a cam bracket 302 fixedly connected to the upper end of the base, wherein a cam 303 is rotatably connected to the cam bracket 302 through a cam 303 shaft arranged in front and rear; a first slide rail 304 fixedly connected to the upper end of the base and located on a left side of the cam bracket 302; a first sliding block 305 slidably connected inside the first slide rail 304, wherein the first sliding block 305 is fixedly connected with one end of a fourth spring 3019, and the other end of the fourth spring 3019 is fixedly connected with the vertical bracket 308 on a left side; a first electric telescopic rod 306, wherein one end of the first electric telescopic rod 306 is fixedly connected to the vertical bracket 308 on a right side, and the other end of the first electric telescopic rod 306 is rotationally connected to the cam 303; a first connecting rod 307, wherein one end of the first connecting rod 307 is rotationally connected to the cam 303, and the other end of the first connecting rod 307 is rotationally connected to the first sliding block 305; two fixed pulleys 3010 connected with the vertical bracket 308 on the left side through first connecting brackets; a second slide rail 3011 fixedly connected to a lower end of the horizontal mounting plate 309; a second sliding block 3012 slidably connected inside the second slide rail 3011, wherein a first processing component 3026 is fixedly connected to a lower end of the second sliding block 3012; a connecting line 3013, wherein one end of the connecting line 3013 is fixedly connected with the first sliding block 305, and the other end of the connecting line 3013 sequentially winds the fixed pulley 3010 at a lower part and the fixed pulley 3010 at an upper part, and finally is fixedly connected with the second sliding block 3012; a first connecting plate 3014 fixedly connected to the vertical bracket 308 on the left side, wherein the first connecting plate 3014 is fixedly connected with a placement frame 3015 for placing a bonesetting plate to be treated; a second vertical connecting rod 3016 fixedly connected to a right side of the second slide rail 3011, wherein the second vertical connecting rod 3016 is slidably sleeved with a connecting block 3018, a lower end of the connecting block 3018 is fixedly connected with a pretreatment spray head 3017, the connecting block 3018 is fixedly connected with a fifth spring 3020, and the other end of the fifth spring 3020 is fixedly connected with the horizontal mounting plate 309; a third connecting rod 3021, wherein one end of the third connecting rod 3021 is rotatably connected with the connecting block 3018, and the other end of the third connecting rod 3021 is rotatably connected with the second sliding block 3012; a housing 3022, wherein a lower end of the housing 3022 is provided with an opening, and the housing 3022 is fixedly connected with the vertical bracket 308 on the right side through a second connecting bracket; a third sliding block 3023 slidably connected inside the housing 3022, wherein sixth springs 3024 are fixedly connected between a lower end of the third sliding block 3023 and a lower end of the housing 3022, and the third sliding block 3023 is in contact with the cam 303; and a fourth connecting rod 3024, wherein a lower end of the fourth connecting rod 3024 is fixedly connected to the third sliding block 3023, an upper end of the fourth connecting rod 3024 is fixedly connected with a first processing plate 3025, and an upper end of the first processing plate 3025 is provided with a plurality of mounting holes.

A working principle and the beneficial effects of the above technical solution are as follows: when dust is removed by a preprocessing component, a processing block is a cleaning block (which may preferably be set in the shape of a drum), a cleaning brush is arranged on the surface of the processing block, the upper surface of the first processing plate may also be provided with a cleaning brush (a cleaning layer), positioning columns are detachably mounted inside a mounting block, the positioning columns are matched with positioning holes or positioning grooves in a bonesetting plate, the sizes and positions of the positioning holes or the positioning grooves in the bonesetting plate can be detected by the positioning columns (when all the positioning columns are inserted into the corresponding positioning holes or positioning grooves, the sizes and positions of the positioning holes or the positioning grooves are correct, preferably, a mounting groove may be formed in the surface of each positioning column, a pressure sensor may be arranged in each mounting groove, and the sizes and positions of the positioning holes or the positioning grooves may be further determined by detection values of the pressure sensors), and the treatment spray head is a dust removal spray head; and

during treatment, the bonesetting plate to be treated is first placed in the placement frame, by starting the first electric telescopic rod, the first electric telescopic rod drives the cam to rotate right and left, the first sliding block is pushed by the first connecting rod to slide left and right

inside the first slide rail, realizing left and right movement of one end of the connecting line connected to the first sliding block (when the sliding block is pulled to the right, a fourth spring is driven to elongate, wherein the fourth spring is arranged to facilitate the connecting line to move to the left under the action of the elastic force), so that the other end of the connecting line moves left and right, and the second sliding block slides left and right within the second slide rail, the second sliding block drives the connecting block to move up and down through the action of the third connecting rod (wherein the fifth spring is arranged so that the connecting block is reliably connected with the horizontal mounting plate, and the second vertical connecting rod is arranged for up and down movement guide), realizing driving of the spray head on the connecting block to move up and down to remove dust from the bonesetting plate to be treated; at the same time, when the cam rotates left and right, the third sliding block is pushed to move up and down inside the housing, the arrangement of the seventh spring makes the connection between the third sliding block and the housing reliable, when the third sliding block moves upward, the processing plate is driven to move upward by the fourth connecting rod, realizing detection of the sizes and positions of the positioning holes or the positioning grooves in the bonesetting plate by the positioning columns, and optionally, a dust removing layer may be arranged on the surface of each positioning column for moving up and down to remove dust from the positioning holes or the positioning grooves; and when the second sliding block moves left and right, the first processing component is driven to move left and right to further remove dust from the surface of the bonesetting plate. The above technical solution can realize the plurality of adjustable processes, and the plurality of functions by one drive of the first electric telescopic rod, and the processing is convenient.

Embodiment 9, on the basis of Embodiment 8, as shown in FIGS 11-12; the first processing component 3026 includes: a hollow fixed connecting block 30261, wherein an upper end of the hollow fixed connecting block 30261 is fixedly connected to the lower end of the second sliding block 3012 through a second electric telescopic rod; a vertical connecting plate 30263 fixedly connected to one side of the hollow fixed connecting block 30261, wherein the vertical connecting plate is also fixedly connected to the second sliding block 3012; a sliding rod 30262, wherein the sliding rod 30262 is slidably connected with a lower end of the hollow fixed connecting block 30261, and a lower end of the sliding rod 30262 penetrates through the hollow fixed connecting block 30261, and one side, close to the vertical connecting plate, of the sliding rod 30262 is provided with a sliding slot; a fourth sliding block slidingly connected inside the sliding slot, wherein the fourth sliding block is connected with one end of an eighth spring 30268, and the other end of the eighth spring 30268 is fixedly connected with the vertical connecting plate ; a disk body 30264 slidably connected with an inner wall of the hollow fixed connecting block, wherein the disk body 30264 is fixedly connected with the sliding rod 30262;

a seventh spring 30265, wherein one end of the seventh spring 30265 is fixedly connected with the disk body 30264, and the other end of the seventh spring 30265 is fixedly connected with the inner wall of the hollow fixed connecting block 30261; and a processing block 30266 connected to the lower end of the sliding rod 30262. A working principle and the beneficial effects of the above technical solution are as follows: the processing block is driven by the second electric telescopic rod to be close to the upper surface of the structure of the bonesetting plate to be treated (e.g., dust removal) or other bone repair instruments, the processing block is close to the upper surface of the bonesetting plate to be treated (e.g., dust removal) or other bone repair instruments under the action of its own gravity, when the upper surface of the setting plate or other bone repair instruments is not in a same straight line, during the left and right movement of the processing block, the processing block moves up and down under the action of the seventh spring, the sliding connection of the sliding rod connected to the processing block is used as a primary movement guide, and the sliding connection of the disk body connected to the sliding rod is used as a secondary guide, so that the processing block of the present invention reliably moves, and facilitates reliable surface treatment of the bone repair instrument.

Obviously, those skilled in the art can make various modifications and variations on the present invention without departing from the spirit and scope of the present invention. Thus, if these modifications and variations of the present invention belong to the scope of the claims of the present invention and their equivalent technologies, the present invention is also intended to contain these modifications and variations.

**Claims**

1. A fracture repair device realizing transition from mechanical fixation (association of osteosynthesis, AO) to biological fixation (biological osteosynthesis, BO), comprising a bone repair instrument (1), **characterized in that** auxiliary components (2) are arranged at positions, close to a repaired bone surface, of the bone repair instrument (1), and each auxiliary component (2) is made of a degradable material.

2. The fracture repair device realizing transition from mechanical fixation (association of osteosynthesis, AO) to biological fixation (biological osteosynthesis, BO) according to claim 1, **characterized in that** the degradable material comprises: a degradable metal material or a composite material thereof or a degradable polymeric material, and the bone repair instrument (1) can be made of biomedical stainless steel, pure titanium, Ti6Al4V or Ti6Al7Nb; and the degradable metal material or the composite material thereof comprises any one of magnesium or a magnesium alloy or a magnesium-based composite, zinc or a zinc alloy or a zinc-based composite, and iron or an iron alloy or an iron-based composite.

3. The fracture repair device realizing transition from mechanical fixation (association of osteosynthesis, AO) to biological fixation (biological osteosynthesis, BO) according to claim 2, **characterized in that**,

   the magnesium or magnesium alloy or magnesium-based composite comprises any one of high purity magnesium, Mg-Zn, Mg-Sr, Mg-Ca, Mg-Li, Mg-Y, Mg-Zn-Ca, WE43, AZ31B, AZ91, HA/Pure Mg, HA/Mg-Zn, HA/Mg-Ca, HA/Mg-Zn-Ca, $\beta$-TCP/Pure Mg, $\beta$-TCP/Mg-Zn, $\beta$-TCP/Mg-Ca, $\beta$-TCP/Mg-Zn-Ca, MgO/Pure Mg, MgO/Mg-Zn, and MgO/Mg-Ca;
   the zinc or zinc alloy or zinc-based composite comprises any one of high purity zinc, Zn-Mg, Zn-Cu, Zn-Ca, Zn-Li, Zn-Y, Zn-Sr, HA/Pure Zn, HA/Zn-Mg, HA/Zn-Cu, HA/Zn-Ca, $\beta$-TCP/Pure Zn, $\beta$-TCP/Zn-Mg, $\beta$-TCP/Zn-Cu, $\beta$-TP/Zn-Ca, ZnO/Pure Zn, and ZnO/Zn-Mg;
   the iron or iron alloy or iron-based composite comprises any one of high purity iron, Fe-X, Fe-Mn-Si, Fe-Mn-C, Fe-Mn-Pd, CNT/Fe, $Fe_2O_3$/Fe, HA/Fe, and $\beta$-TCP/Fe, wherein X is any one of Mn, Co, Al, W, Pt, Ag, Sn, B, C, and S; and
   the degradable polymeric material comprises any one of polylactic acid, a copolymer, polycaprolactone, polydioxanone, polyhydroxyalkanoate, polytrimethylene carbonate, polyurethane, and polyether urethane.

4. The fracture repair device realizing transition from mechanical fixation (association of osteosynthesis, AO) to biological fixation (biological osteosynthesis, BO) according to claim 1, **characterized in that** the auxiliary components (2) comprise any one or more of a pad, a plate, and a block, wherein the pad, the plate, and the block comprise any one or more of a circular ring-shaped gasket (3), a plate-shaped washer (4), and a square cushion block (5).

5. The fracture repair device realizing transition from mechanical fixation (association of osteosynthesis, AO) to biological fixation (biological osteosynthesis, BO) according to claim 1, **characterized in that** the surface of each auxiliary component (2) is subjected to surface treatment comprising any one of micro-arc oxidation, chemical deposition, and anodization.

6. The fracture repair device realizing transition from mechanical fixation (association of osteosynthesis, AO) to biological fixation (biological osteosynthesis, BO) according to claim 1, **characterized in that** a connection mode between the auxiliary components (2) and the bone repair instrument (1) comprises direct contact connection or pin connection.

7. The fracture repair device realizing transition from mechanical fixation (association of osteosynthesis, AO) to biological fixation (biological osteosynthesis, BO) according to claim 1, **characterized by** further comprising a mounting device, wherein the mounting device comprises:

a first screw (6), wherein the first screw (6) is disposed above the bone repair instrument (1), the first screw (6) is a double-headed screw, two ends of the first screw (6) are oppositely threaded, and one end of the first screw (6) is provided with a first rocker (7);

moving plates (8), wherein the two moving plates (8) are symmetrically disposed on two sides of the bone repair instrument (1), the two moving plates (8) are respectively threadedly connected with two ends of the first screw (6), and a plurality of rollers (9) are arranged on one side, facing the bone repair instrument (1), of each moving plate (8);

a guide plate (10), wherein the guide plate (10) is disposed at one ends, away from the first screw (6), of the moving plates (8), two ends of the guide plate (10) respectively penetrate through the moving plates (8) on both sides, and are slidably connected with the moving plates (8), two ends of the guide plate (10) each are provided with a baffle (11), a first spring (12) is disposed between each baffle (11) and the corresponding moving plate (8), one end of each first spring (12) is fixedly connected with a side wall of the corresponding moving plate (8), and the other end of each first spring (12) is fixedly connected with a side wall of the corresponding baffle (11);

a first rotary shaft (13), wherein the first rotary shaft (13) is disposed above the guide plate (10), the first rotary shaft (13) is perpendicular to the bone repair instrument (1), one end of the first rotary shaft (13) is rotatably connected to an upper surface of the guide plate (10), the other end of the first rotary shaft (13) is provided with a gear (14), an upper surface of the gear (14) is provided with a fixed column (24), a first rack (15) is arranged on a left side of the gear (14), the first rack (15) is meshed with the gear (14), one end, away from the gear (14), of the first rack (15) penetrates through the moving plate (8) at an upper part, and is slidably connected with the moving plate (8), one side, away from the guide plate (10), of the first rack (15) is provided with a first clamp plate (16), a second spring (17) is arranged between the first clamp plate (16) and the moving plate (8), one end of the second spring (17) is fixedly connected to a side wall of the first clamp plate (16), the other end of the second spring (17) is fixedly connected with a side wall of the moving plate (8), a second rack (18) is arranged on a right side of the gear (14), the second rack (18) is meshed with the gear (14), one end, away from the gear (14), of the second rack (18) penetrates through the moving plate (8) at a lower part, and is slidably connected with the moving plate (8), one side, close to the guide plate (10), of the second rack (18) is provided with a second clamp plate (19), a third spring (20) is arranged between the second clamp plate (19) and the moving plate (8), one end of the third spring (20) is fixedly connected with a side wall of the moving plate (8), and the other end of the third spring (20) is fixedly connected with a side wall of the second clamp plate (19); and

second screws (21), wherein the two second screws (21) are symmetrically disposed on both sides of the bone repair instrument (1), each second screw (21) is threadedly connected with the middle of the corresponding moving plate (8), one end of each second screw (21) is provided with a second rocker (22), the other end of each second screw (21) is provided with a pressing plate (23), and the pressing plates (23) are in contact with a side wall of the bone repair instrument (1).

8. The fracture repair device realizing transition from mechanical fixation (association of osteosynthesis, AO) to biological fixation (biological osteosynthesis, BO) according to claim 1, **characterized by** further comprising:

an image acquisition device, disposed above the bone repair instrument (1), and configured to acquire an image that the auxiliary components (2) are mounted on the bone repair instrument (1), and generate a first image;

a processing device connected to the image acquisition device, wherein the processing device performs processing on the first image to equally divide the first image into a plurality of actual image regions;

a controller connected with the processing device, and configured to compare the first image with a preset standard image, and generate a comparison result;

an alarm electrically connected to the controller; wherein

the controller controls the operation of the alarm based on the comparison result, which comprises the following steps of

step 1: calculating an offset obtained by comparing the first image with a preset standard image by a formula (1):

$$\varphi = \frac{\pi}{\sqrt{e*(1+e)^2}} * \sum_{i=1}^{N}(f_i - g_i)^2 *(f_i + g_i) \quad (1)$$

wherein $\varphi$ is an offset obtained by comparing the first image with a preset standard image, e is a constant, e is 2.72, $\pi$ is a constant, $\pi$ is 3.14, N is the total number of the actual image regions in the first image, $f_i$ is a gray value of an ith actual image region in the first image, and $g_i$ is a gray value of an ith preset standard image region in the preset standard image;

step 2: calculating an actual similarity between the first image and the preset standard image by a formula (2):

$$simY = \varphi * \frac{\sum_{i=1}^{N}(f_i * g_i)}{\sqrt[4]{\sum_{i=1}^{N}f_i^2 * \sum_{i=1}^{N}\left(\frac{g_i}{f_i}\right)^2}} * \frac{1}{N}$$

wherein simY is an actual similarity between the first image and the preset standard image; and

step 3: comparing, by the controller, the actual similarity between the first image and the preset standard image with a preset similarity, and when the actual similarity is less than the preset similarity, controlling, by the controller, the alarm to give an alarm.

9. The fracture repair device realizing transition from mechanical fixation (association of osteosynthesis, AO) to biological fixation (biological osteosynthesis, BO) according to claim 1, **characterized in that** the bone repair instrument (1) comprises a bonesetting plate, and the fracture repair device further comprises: a pretreatment device (30), wherein the pretreatment device (30) comprises:

a fixed base (301), wherein left and right sides of an upper end of the fixed base (301) are fixedly connected to a horizontal mounting plate (309) through vertical brackets (308);

a cam bracket (302) fixedly connected to the upper end of the base, wherein a cam (303) is rotatably connected to the cam bracket (302) through a cam (303) shaft arranged in front and rear;

a first slide rail (304) fixedly connected to the upper end of the base and located on a left side of the cam bracket (302);

a first sliding block (305) slidingly connected inside the first slide rail (304), wherein the first sliding block (305) is fixedly connected with one end of a fourth spring (3019), and the other end of the fourth spring (3019) is fixedly connected with the vertical bracket (308) on a left side;

a first electric telescopic rod (306), wherein one end of the first electric telescopic rod (306) is fixedly connected to the vertical bracket (308) on a right side, and the other end of the first electric telescopic rod (306) is rotationally connected to the cam (303);

a first connecting rod (307), wherein one end of the first connecting rod (307) is rotationally connected to the cam (303), and the other end of the first connecting rod (307) is rotationally connected to the first sliding block (305);

two fixed pulleys (3010) connected with the vertical bracket (308) on the left side through first connecting brackets;

a second slide rail (3011) fixedly connected to a lower end of the horizontal mounting plate (309);

a second sliding block (3012) slidably connected inside the second slide rail (3011), wherein a first processing component (3026) is fixedly connected to a lower end of the second sliding block (3012);

a connecting line (3013), wherein one end of the connecting line (3013) is fixedly connected with the first sliding block (305), and the other end of the connecting line (3013) sequentially winds the fixed pulley (3010) at a lower part and the fixed pulley (3010) at an upper part, and finally is fixedly connected with the second sliding block (3012);

a first connecting plate (3014) fixedly connected to the vertical bracket (308) on the left side, wherein the first connecting plate (3014) is fixedly connected with a placement frame (3015) for placing a bonesetting plate to be treated;

a second vertical connecting rod (3016) fixedly connected to a right side of the second slide rail (3011), wherein the second vertical connecting rod (3016) is slidably sleeved with a connecting block (3018), a lower end of the connecting block (3018) is fixedly connected with a pretreatment spray head (3017), the connecting block (3018)

is fixedly connected with a fifth spring (3020), and the other end of the fifth spring (3020) is fixedly connected with the horizontal mounting plate (309);

a third connecting rod (3021), wherein one end of the third connecting rod (3021) is rotatably connected with the connecting block (3018), and the other end of the third connecting rod (3021) is rotatably connected with the second sliding block (3012);

a housing (3022), wherein a lower end of the housing (3022) is provided with an opening, and the housing (3022) is fixedly connected with the vertical bracket (308) on the right side through a second connecting bracket;

a third sliding block (3023) slidably connected inside the housing (3022), wherein sixth springs (3024) are fixedly connected between a lower end of the third sliding block (3023) and a lower end of the housing (3022), and the third sliding block (3023) is in contact with the cam (303); and

a fourth connecting rod, wherein a lower end of the fourth connecting rod is fixedly connected to the third sliding block (3023), an upper end of the fourth connecting rod is fixedly connected with a first processing plate (3025), and an upper end of the first processing plate (3025) is provided with a plurality of mounting holes.

**10.** The fracture repair device realizing transition from mechanical fixation (association of osteosynthesis, AO) to biological fixation (biological osteosynthesis, BO) according to claim 9, **characterized in that** the first processing component (3026) comprises:

a hollow fixed connecting block (30261), wherein an upper end of the hollow fixed connecting block (30261) is fixedly connected to the lower end of the second sliding block (3012) through a second electric telescopic rod (30267);

a vertical connecting plate (30263) fixedly connected to one side of the hollow fixed connecting block (30261);

a sliding rod (30262), wherein the sliding rod (30262) is slidably connected with a lower end of the hollow fixed connecting block (30261), and a lower end of the sliding rod (30262) penetrates through the hollow fixed connecting block (30261), and one side, close to the vertical connecting plate (30263), of the sliding rod (30262) is provided with a sliding slot;

a fourth sliding block slidingly connected inside the sliding slot, wherein the fourth sliding block is connected with one end of an eighth spring (30268), and the other end of the eighth spring (30268) is fixedly connected with the vertical connecting plate (30263);

a disk body (30264) slidably connected with an inner wall of the hollow fixed connecting block (30261), wherein the disk body (30264) is fixedly connected with the sliding rod (30262);

a seventh spring (30265), wherein one end of the seventh spring (30265) is fixedly connected with the disk body (30264), and the other end of the seventh spring (30265) is fixedly connected with the inner wall of the hollow fixed connecting block (30261); and

a processing block (30266) connected to the lower end of the sliding rod (30262).

FIG. 1

FIG. 2

FIG. 3

FIG. 4

FIG. 5

FIG. 6

FIG. 7

FIG. 8

FIG. 9

FIG. 10

FIG. 11

EP 4 260 826 A1

FIG. 12

FIG. 13

26

FIG. 14

L. Tian et al. / Biomaterials 180 (2018) 173–183                                                                     181

FIG. 15

Hydrogen evolution per unit time of magnesium alloys

FIG. 16

Accumulated hydrogen evolution of magnesium alloys

FIG. 17

FIG. 18

**Ion concentration in corresponding simulated body fluid after corrosion of different magnesium/zinc alloys for a period of time(mg/L)**

| Ion / Sample | $Mg^{2+}$ | $Zn^{2+}$ | $Ca^{2+}$ | $Al^{3+}$ | $P^{3+}$ |
|---|---|---|---|---|---|
| Pure Mg | 430.953 | 0 | 73.113 | 0.509 | 0.392 |
| WE43 | 399.157 | 0 | 75.888 | 0.803 | 0.617 |
| AZ91 | 351.855 | 0 | 110.050 | 1.071 | 8.783 |
| Mg-Zn-Ca | 352.765 | 0.199 | 107.164 | 0.695 | 14.008 |
| Mg-Zn-Ca-Y | 337.125 | 0 | 120.089 | 0.840 | 21.556 |
| Pure Zn | 38.530 | 6.552 | 53.681 | 0.235 | 15.957 |
| Zn-1Mg | 36.103 | 4.998 | 75.377 | 0.227 | 14.064 |
| Blank | 35.659 | 0.069 | 81.367 | —— | 35.837 |

FIG. 19

| Sample / Corrosion rate | Pure Mg | WE43 | AZ91 | Mg-Zn-Ca | Mg-Zn-Ca-Y | Pure Zn | Zn-1Mg |
|---|---|---|---|---|---|---|---|
| $\Delta m1$ | 0.929 | 0.957 | 0.973 | 0.965 | 1.003 | 4.042 | 3.729 |
| $\Delta m2$ | 0.369 | 0.419 | 0.794 | 0.813 | 0.847 | 3.996 | 3.659 |
| $\Delta m=\Delta m1-\Delta m2$ | 0.56 | 0.538 | 0.179 | 0.152 | 0.156 | 0.046 | 0.07 |
| T/h | 47.5 | 47.5 | 47.5 | 46.5 | 46.5 | 351 | 351 |
| $C=K\Delta m/\rho At$ (mm/a) | 879 | 845 | 281 | 244 | 250 | 2.38 | 3.62 |

FIG. 20

# EP 4 260 826 A1

| INTERNATIONAL SEARCH REPORT | International application No. |
|---|---|
| | **PCT/CN2021/100712** |

**A.    CLASSIFICATION OF SUBJECT MATTER**

A61B 17/80(2006.01)i;  A61L 31/02(2006.01)i;  A61L 31/06(2006.01)i;  A61L 31/14(2006.01)i

According to International Patent Classification (IPC) or to both national classification and IPC

**B.    FIELDS SEARCHED**

Minimum documentation searched (classification system followed by classification symbols)

A61B 17;A61L 31

Documentation searched other than minimum documentation to the extent that such documents are included in the fields searched

Electronic data base consulted during the international search (name of data base and, where practicable, search terms used)

CNABS, VEN, CNKI, CNTXT: 中国人民解放军总医院, 北京市春立正达医疗器械, 彭江, 王振国, 孟昊业, 卢强, 潘超, 汪爱媛, 周皓, 许文静, 刘伟, 张星燕, 可降解, 可吸收, 安装, 预处理, 骨, 安装, 螺杆, 导向板, 移动板, 固定底座, 凸轮支架, 滑轨, bone, plate?, absorb+, degradabl+, biodegradabl+

**C.    DOCUMENTS CONSIDERED TO BE RELEVANT**

| Category* | Citation of document, with indication, where appropriate, of the relevant passages | Relevant to claim No. |
|---|---|---|
| PX | CN 112617995 A (CHINESE PLA GENERAL HOSPITAL; BEIJING CHUNLIZHENGDA MEDICAL INSTRUMENTS CO., LTD.) 09 April 2021 (2021-04-09)<br>  claims 1-10 | 1-10 |
| X | CN 103908328 A (THE CHINESE UNIVERSITY OF HONG KONG) 09 July 2014 (2014-07-09)<br>  description paragraphs [0023]-[0049] and figures 1A-6B | 1-6 |
| A | CN 103908328 A (THE CHINESE UNIVERSITY OF HONG KONG) 09 July 2014 (2014-07-09)<br>  description paragraphs [0023]-[0049] and figures 1A-6B | 7-10 |
| X | CN 108338828 A (XINJIANG UNIVERSITY) 31 July 2018 (2018-07-31)<br>  description, paragraphs [0045]-[0070] and figures 1-3 | 1-6 |
| X | US 2016331423 A1 (BIOTRONIK AG) 17 November 2016 (2016-11-17)<br>  description, paragraphs [0032]-[0040] and figure 1 | 1-6 |
| X | JP 5145375 B2 (TAKIRON K. K. ET AL) 13 February 2013 (2013-02-13)<br>  description, abstract | 1-6 |

☑ Further documents are listed in the continuation of Box C.        ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **25 August 2021** | **03 September 2021** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

International application No.

**PCT/CN2021/100712**

### C. DOCUMENTS CONSIDERED TO BE RELEVANT

| Category* | Citation of document, with indication, where appropriate, of the relevant passages | Relevant to claim No. |
|---|---|---|
| X | CN 106725785 A (SHANGHAI SHIBIKANG MEDICAL INSTRUMENT CO., LTD.) 31 May 2017 (2017-05-31)<br>description, paragraphs [0042]-[0047] and figures 1-5 | 1-3, 5-6 |
| X | CN 203328788 U (THE CHINESE UNIVERSITY OF HONG KONG) 11 December 2013 (2013-12-11)<br>description paragraphs [0043]-[0048] and figures 1A-6B | 1-6 |
| X | CN 110585490 A (SHANGLI INTERNATIONAL STANDARDS CO., LTD.) 20 December 2019 (2019-12-20)<br>description, paragraphs [0021]-[0032] | 1-3 |
| A | CN 110897699 A (SHENZHEN AISI TECHNOLOGY CO., LTD.) 24 March 2020 (2020-03-24)<br>entire document | 1-10 |
| A | CN 105455887 A (TIBET DEKANG MEDICAL DEVICES CO., LTD.) 06 April 2016 (2016-04-06)<br>entire document | 1-10 |

Form PCT/ISA/210 (second sheet) (January 2015)

INTERNATIONAL SEARCH REPORT
Information on patent family members

International application No.

**PCT/CN2021/100712**

| Patent document cited in search report | | | Publication date (day/month/year) | Patent family member(s) | | | Publication date (day/month/year) |
|---|---|---|---|---|---|---|---|
| CN | 112617995 | A | 09 April 2021 | None | | | |
| CN | 103908328 | A | 09 July 2014 | None | | | |
| CN | 108338828 | A | 31 July 2018 | CN | 108338828 | B | 16 March 2021 |
| US | 2016331423 | A1 | 17 November 2016 | EP | 3093030 | A1 | 16 November 2016 |
| | | | | US | 10278753 | B2 | 07 May 2019 |
| JP | 5145375 | B2 | 13 February 2013 | JP | 2010227585 | A | 14 October 2010 |
| CN | 106725785 | A | 31 May 2017 | CN | 106725785 | B | 06 December 2019 |
| CN | 203328788 | U | 11 December 2013 | None | | | |
| CN | 110585490 | A | 20 December 2019 | None | | | |
| CN | 110897699 | A | 24 March 2020 | CN | 110897699 | B | 22 September 2020 |
| CN | 105455887 | A | 06 April 2016 | None | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• CN 202011460429